# EUROPEAN PATENT SPECIFICATION

(11) **EP 1 137 791 B1**
(45) Date of publication and mention of the grant of the patent: **18.03.2009**
(21) Application number: 99963487.6
(22) Date of filing: 09.12.1999
(51) Int. Cl.: C12N 15/85, C12N 15/13, C12N 5/10, A01K 67/027, C07K 14/47

(54) **REGULATORY SEQUENCES OF THE MOUSE VILLIN GENE - USE IN TRANSGENESIS**
REGULIERENDE SEQUENZEN DES VILLIN-GENS AUS DER MAUS UND VERWENDUNG ZUR HERSTELLUNG VON TRANSGENEN TIEREN
REGIONS REGULATRICES DU GENE VILLIN DE LA SOURIS - LEUR UTILISATION DANS LA TRANSGENESE

(30) Priority: 09.12.1998 WO PCT/EP98/08009
(43) Date of publication of application: 04.10.2001
(60) Divisional application: 06023613.0
(73) Proprietor: Centre National de la Recherche Scientifique, 75794 Paris Cedex 16 (FR); Institut Curie, 75248 Paris Cedex 05 (FR)
(72) Inventor: PINTO, Daniel, F-93170 Bagnolet (FR); ROBINE, Sylvie, F-92170 Vanves (FR); JAISSER, Frédéric, F-92240 Malakoff (FR); LOUVARD, Daniel, F-92330 Sceaux (FR); NIEWÖHNER, Jens, D-81477 München (DE)
(74) Representative: Desaix, Anne
(86) International application number: PCT/EP1999/009782
(87) International publication number: WO 2000/034493

(56) References cited:
- EP-A- 0 496 174
- DUNBAR L ET AL: "Functional analysis of the mouse villin gene promoter" MOLECULAR BIOLOGY OF THE CELL, vol. 9, no. Suppl, November 1998 (1998-11), page 1840 (317A) XP002113601
- ROBINE S ET AL: "Epithelial cell growth and differentiation. IV. Controlled spatiotemporal expression of transgenes: new tools to study normal and pathological states" AM J PHYSIOL, vol. 273, no. 4 (Pt1), - October 1997 (1997-10) pages G759-G762, XP002113602 cited in the application
- ROBINE S ET AL: "Gene targeting in epithelial cells of the endodermal cell lineage using the human villin promoter" CELL BIOLOGY INTERNATIONAL, vol. 18, no. 5, 1994, page 471 XP002113603
- PINTO D ET AL: "Regulatory sequences of the mouse villin gene that efficiently drive transgenic expression in immature and differentiated epithelial cells of small and large intestines" JOURNAL OF BIOLOGICAL CHEMISTRY., vol. 274, no. 10, 5 March 1999 (1999-03-05), pages 6476-6482, XP002113604
- PRINGAULT E ET AL: "Structure of the human villin gene" PROCEEDINGS OF THE NATIONAL ACADEMY OF SCIENCES OF USA., vol. 88, no. 23, 1 December 1991 (1991-12-01), pages 10811-10815, XP002113605 cited in the application
- ROBINE S ET AL: "Regulatory sequences on the human villin gene trigger the expression of a reporter gene in a differentiating HT29 intestinal cell line" JOURNAL OF BIOLOGICAL CHEMISTRY., vol. 268, no. 15, 25 May 1993 (1993-05-25), pages 11426-11434, XP002113606 cited in the application
- TREMP G ET AL: "Induction of a lesion resembling human thymoma in transgenic mice" PROC ANNU MEET AM ASSOC CANCER RES, vol. 34, 1993, page A3180 XP002113607

### SEQUENCE LISTING

<110> CENTRE NATIONAL DE LA RECHERCHE SCIENTIFIQUE INSTITUT CURIE
<120> REGULATORY SEQUENCES OF THE MOUSE VILLIN GENE - USE IN TRANSGENESIS.
<130> 4121A _AD
<140> PCT/EP 99/09782
   <141> 1999-12-09
<150> PCT/EP 98/08009
   <151> 1998-12-09
<160> 20
<170> PatentIn Ver. 2.1
<210> 1
   <211> 8995
   <212> DNA
   <213> mouse
<220>
   <221> intron
   <222> (3489)..(8981)
<220>
   <221> exon
   <222> (3443)..(3487)
   <223> exon 1
<400> 1
   gatctggtgc accaaggaca ctgtggtccc agcactgggg aggtggaggg aggagggtca 60
   gaagtttaag gtcatccttg gttacatagc aaggtttcag ccagcttcag ctacatgaaa 120
   cctttgtttg tttgtttgtt tgttttaaag cattaataaa taataccata aggaggttgg 180
   cagtggtggc agacaccttt aattccagta ttcaggaggc agaagcaggc agatctctgt 240
   gagttcgaag tcagcctagt ctgcaaagct agttccagga tggcaagggc tacacagaga 300
   aaccttgtct cataaaacca aagtagtagt agtagtagta atgccataga gaaaattgga 360
   gtccattcag gatggaccat cctataagat gattctcttg acccaggtaa gctaatgtca 420
   tggggaaagg ggatgggact gtcctagatt aaaaagtgct gaggcgatgc ctattctcaa 480
   tttgattcca tatgaaaagg ctgataaggc ccaagagaag tggaactggg actctggact 540
   gaagacgtga cggccttata aacactggca cttataaaca cttataaaca ctggcacagg 600
   cgttcaggtt tgaagatcac tttcaaacca cagaacagaa agtgctcgct cgtcctcagc 660
   gtagcgagca ctggctgcag aagagtgata tttagtgaaa gctaccttca caatatcttt 720
   gcacttatca catacacgtg tcaaatgtgc taactcccta gtccacagat ggctgttaca 780
   ctcgtttctg ctttcccatc tggttgacat ttgtcagaac cagaaattag aaatgtgggt 840
   atttatttgt gtgctgagga caccatccag ggcttttcac atttcaggca catggtttac 900
   taactgggct acttctccaa cggtttgaaa ccatttgttt tatatttact tattttgtgt 960
   gcatgaggta ggcatgtata cgtatgtata ggagtcatgc atgtggctgc taccctcaaa 1020
   atcattgcag atccccagca agtgaagtca ccgagcgttg taagttgtta tgtgggactg 1080
   ggagccaagg ctgggttctc tgcaagagca gccagtggcc ttaaccatgg gaccagctct 1140
   ctaggcctaa ggtaatcttt agttttttaa aaatatatat tctcagccgg gtgtggtggc 1200
   acacgccttt aatcccagca cttgagaggc tgaggtgtag gaattataca cacaggccag 1260
   ctggggtgca gagcttggcc ctgttttttt tgttttttct ttatgtgcac tggtgtctta 1320
   cctgcgtgta tgtccgtgca agggtgtcag atcccttgga gctggagtta aagacagttg 1380
   tgatcacgct gccgttacag atgctggaaa ttgaacccag gtgtccctag agaagcagcc 1440
   agtgctctta acttctgagc cacccctcca accctgcttt tagagactct taaccttttg 1500
   tgtaatgtgg gaactgagtg gatcttgcac ttaccaagtg tgtgctgcgc tgtagcatca 1560
   ctgagcccgt acccacacga ctagtggata cagtttaagg gcaaacactt aacaatgaca 1620
   atagttggat agagtttgaa tatagtcctg agctattggt tagcgtgacc tttgctgtcc 1680
   ttagcatgtg ctgtgagaag atagaaaaat gaagacttga gtctagtcct ggaacccaca 1740
   gaggcaggcg agaacccact cctgaaagtt gttctctgag cttcacatac aacttcacat 1800
   aatagttaca atgataataa taattagtaa attcttttaa aaggtatatg ttgggaggga 1860
   gagatggctc agcttccagg agcacttgct gctcttgcag aggacctaga ttcagttccc 1920
   aggactcata tggtggctca cagccatctg taaatccagt tccagagggt tccacaccct 1980
   cttctggcct ccacaggcac cacatacata gtacacagac atacatgcag gcaaaacacc 2040
   catacacaca taaataaata aggaaactta aaaggtgcat gtgttggtaa acattgtgct 2100
   tacacatgct gattgaagac atgtacaacg cacacactga agagggatct ggggctggag 2160
   agatggctca gcggttaaga gcactgactg ctcttccgaa ggaaggtcct gagttcaaat 2220
   cctagcaacc acatggtggc tcacaaccat ccataatgag atctgacacc ctcttctggt 2280
   gcatctgaag acagctgcag agctacagtg tacttagata tactaataaa taaatctttt 2340
   tttaaaaaaa tgaagaggga tctgagacac ctcaaaagag attatgagca gtgactcacg 2400
   ggtgattatc tatcctggag tttttccttt ccgcttggct tgcaactggg tggacagacg 2460
   ccccttttca ttcacaagaa cgggtgctac attatttctg aacaaaacag cacctgcagt 2520
   atgtttactg tccttgctga ctatgagcac gcgcacgcgc gcgcgcacac acacacacac 2580
   acacacacac acacacacac acacacacac attcagtctc cagagctctt gggaaggtca 2640
   agaagaggct gccctcaaac acgatcttca tctttccctc ctaaaggaga ccacgattcc 2700
   aaggtggcag aagatctaca gggggcagag gcagggaggg ggaagcaggc catggtttcc 2760
   agagacctac agcagagggc agcaaggcag atccccaggt ccagggcagg gaggtggagg 2820
   cccttgttcc gaggagaagg caggcggcag aacagggttc aaaggcacag gtttatggca 2880
   gctcataaaa gtggaggtcg tggctcactc agaaaggagg aagaagggaa aggcccttgt 2940
   gcccactgag cgagggtcat gctgagtagg agagatctgc aggggtgcca ggagccccac 3000
   ctgtctgtcc caagggaacc ccaagtgtga actctggcct tgggtgctga gttccagcta 3060
   caagacccca ggagtcctac tccatcccca tccagtgccc cctcgccccg ccacacccca 3120
   cccccgactc ccgtgccact tctctagggc tggagggtgg ccagccctgg tgggggttgc 3180
   ctacctgcag gtagagccca ggtcctagcc ggaagtgcac cccatccctg aagctgcaga 3240
   gccaagggcg gggcacacgg cagctcaggc tgtcaggctg ttgctgggct ctaggttccc 3300
   agggacctgg gcacctactt ccccaccccc ccatccattc tctctggggc cctatcttcc 3360
   cttatatggt gaaggaagtt cctggggggg gggggtggtg gtgaggacaa aggtcgttcg 3420
   gtctcctgca gccagcttgc ca caa ctt cct aag atc tcc cag gtg gtg gct 3472
   gcc tct tcc aga cag gtaaggcaat tgggtgggga cacatggtga ccacaggtgg 3527
   ttggagggga cagggtcctt gcttctctct ggcagcctgt gctttctgta gcaccttggt 3587
   ataagtttgg gggtgaggta aggtgctctg aaactctgaa agaagcaaga agccagcagg 3647
   ctgtcttggg ccttcaatga aggaagttca cagaccccct ttcctgtaag tcaccttcgc 3707
   ttcatctgtg tagattccct gggaccaagg tggctcctgg gactcagatt tctacaatta 3767
   aaatcaggac agtcctgaga cttggactcc gtgcctgtat ttactacttc tctctggctg 3827
   ctcatttctg tgttcatgtc ttacacatct gaaatggttt ctttgtgtca ccattcccct 3887
   gacactcctg ggaggtcgta tccttggcac atgtatcctg ggatgtaagc tgcagccacc 3947
   aggagagagg gggagagtca ggagctgtgt cctaggccct attaggcctg gacatcaccc 4007
   ctttcctaga aatggcccct ccatttttcg gttaccatga tctattttat atcagagtgg 4067
   gcagtgaaag ccaaacctgc ccagaagttt gggactcact cagaccaagg ttatctgctc 4127
   agaaatcccc ctgtcacttg aggttgggag aatctgcctc tgggggcttc caggtcttgg 4187
   ttagcaggag ggtatccttt gtatagggca tgacctagtc tatggtgtta ctacattcct 4247
   gtccagttaa aagctggaac taaaacccac ggcagcgccc aggattctct acagttgtac 4307
   cccaagaaca acaagacagt agatatgcaa ggataggtag ctggggagaa gaagaactta 4367
   aaccccccca aaggcccaca ggttccgttc cctagttcac aatgccagta tgagtgctag 4427
   ctactatggg ctgtgagttg gtagctacaa gcatgagtga tgttcatgtg tgtagtgtgt 4487
   ataatctgag cacttgggag gctgaagcag gaggattgct atatgtttga ggccagcctg 4547
   agctatagag cgagactttg tctttaagaa aaaaatgaaa gcccagcagt ggtggcacac 4607
   gcctttaatc ccagcacttg ggaggcagaa gcaggcagat ttctgagttc aaggccagcc 4667
   tggtctatag agtgagttcc aggacagcca gggctacaca gagaaaccct gttttgaaaa 4727
   accagaaaaa caaaacaaaa caaaacaaaa caaaacccaa acccaaaccc aaacctctca 4787
   tctctcatct ctctaggctg tgtctgtcta ggtggtagag tttggggact tcagacttat 4847
   atataaatag gcctttttat cactggtcag agacgagaaa ggtttcagtc tgggacacag 4907
   tgggaccctg agaaagtact ccttgccagc ccaaaaattc tgggaaggct tcctggagga 4967
   agtgtgtccc gatcagacta ctgttctaga aggcagaaga gagggttgga agaatgttgg 5027
   tggacagaca gttggaacag aaggacagga gggggaggca tccaagattc tgaacatgta 5087
   gctgactttt ggttctctgg gtgacaagtg tcccccaggg atagggctgt agaaagggga 5147
   ccaggggtga gccaatgagt tcaagttgag ggacacatcc agcccagggt ccttgctggc 5207
   aagctaaaga atgagagccc tctaaccctc cctgaagttt aggggagaca ggagagctga 5267
   ggagatcctt ctagggtgaa ggagaggtat ctgctctgac caacatggct aggagcagaa 5327
   gcagttggac cagttacccc tcagaaccag ccatcccctc ttggctctaa ggaggctggg 5387
   cccctttctg tttaagaatc ttacttttct tcagagagag gcagcaagcc tttgtcccct 5447
   ccctgttggt caataaacac ccctgtgtgt aacattagtt tattttactg tcagtttgct 5507
   ccaggacagt ccatctggta gacctctgct cctaactcac caaggtatgg cccacattcc 5567
   tcacccagaa gagtgcagaa gagagcctta gagaaagggt aacagtaaca aagatggcca 5627
   gaataaaaca aaaactacta tcctttgtac ccaaattggt tttgctgaac caggaggggg 5687
   tgtgtgagtg tatgtgtgtg tgtgtgtgtg tgtgtgtgtg tgtgtgtgtg tgtgtgtgtg 5747
   tgtgtgtgtg tgtcttgggg gacttttcat gctaaagaat atctgatatt ggcgcccatg 5807
   ccaacagggg tattggggag agtcaggctt ctgcaaacac agtaagctgc ccaagatgga 5867
   ttggtggcct gaatcaccaa ggggcaggct gatcagagtg gacagaacat cacaagataa 5927
   gccaccctgt ggggctcaga agagggagtt tacaagaggt aaaggccaag ccatttatta 5987
   tccaagacat gactcaaaat caaagtgcaa ggagagatta gctggagaga tggggctgtc 6047
   agtgtgggac acctgacctt gcacttatta gtcactaggc caaggagcag tcacagaggg 6107
   tgactgggtc ctactcagct tggagcaggc acgtggagaa tgggtgacct ccatcctgat 6167
   ggagagggct gagcaccacc aggtacaagt gttccctgtg tctcatgcca ggattcctgg 6227
   ccagttttca aaggactaag gactcatctc tggtggaaac aaagtatcca agccctaagc 6287
   cccattttgg tctaattaaa tcagaacccc tggggatgca ggctctgagc agcaggagct 6347
   ttttaaaaag ctcccaggtg attctgatca gcagctggaa caaacacagc tacaggttca 6407
   aacagaaaga ggcaaagcta gggaaagctt gggatgggga gccttcttcc aggccagtag 6467
   atggaggctg gttagcagtg gtggcagctt ctctctgcct gtcatatagc tatccatcca 6527
   ctcatccatc catacaccca cccatccatt tatgcaccca tccttccatc catccatcta 6587
   tccagctacc cacccacgca tccatccaaa ccttcctttt ctccttcttt ctttcttttt 6647
   tccttcactc attcatttat ccaacagaga actggtattg tactaaatgt gggagattta 6707
   attaattttt agaagctctg ttgattgact gattgtgcat gtatgtggac aggtacatac 6767
   cacagcacac gtgtggcaat cggagaaagg ttttgggtgt tgttttctct tcccaccgtg 6827
   tgggttctgg ggattgaact caaattatcg ggctggtggc aagtgtcttt accaccgagc 6887
   cattttgctg acacatcatt attattagaa agcatcttat gtagtccagg ctggcctcaa 6947
   gcttgctatg tcgccacgga tgacctttaa ctcctgctct tccagcctcc acccgagtgc 7007
   taggtttaca ggtgttcaac tggtgaatgc ctttaatccc agcactctgt gggggggggg 7067
   ggggaggcgg atccctgagt tggaggccag tttggtctac agagtttcag gatacctggg 7127
   gctatacagg gaaaccctat cccaaacaaa caaacaaaca aacaaaaaat attctgtgca 7187
   ataatcacag agattagagg atattagtag ggtagtaggg ctggtgaggg agagtcatgc 7247
   tttcttttgt attataatag taaagtactc acaagatgca ttatctatct atctatctat 7307
   ctatctatct atctatctat ctatctacct acctacctac ctatccatcc atccatctat 7367
   cgtatagccc aggctgcttt gactctgaat gctcctattt ctgggtcaac tcttcacccc 7427
   tagtgttggg tttaccaaca cccagacatt tattttattt tgttttattt tattaatcta 7487
   ggagctcagg gtgggactca gggtcttgtg catgctaagc aagctctctg ccacagagct 7547
   gcagctccag tccccatttt gttcaggtga ctctgtgaca gttgtcatat tcgcagcgct 7607
   atgtagctct ctccacctcc cagttccagc actttctggt catcccagtg ggcgggcaac 7667
   tctgtgctca ccagtgccct gttccctgtc ttcagaccta catatttgcc tgtctgaaca 7727
   gttcatgtaa atgggatgcg ttcctgtgta ttcttttatg gctggcccct ttatcttagc 7787
   acagtttgtg ttgggccatg tgtcactgct atactctatc ttatcatcat cttatggctt 7847
   aatagtgttc ctttgtgtgg ataaaccact ttctgtttca tttactgatg gaaatttgtg 7907
   gccccacccc cacccttttt ttttttattt gagacaaggt ctttctgtgt aatcttgcaa 7967
   tcttggctgt cctgagctca ctctgtagac caggctgtga ggctgtcctt ccacttttga 8027
   cactcctgtg aacagagtag ccatgaactt caaagacaat tttctgtttt ggtttgtttt 8087
   ttacatttgt gtgtgtatgc gtgtatatgt gcatgtttgt gtcttcaggt gctcacatgt 8147
   gtgtacctgt gtgtgggaca gagaacaaac cgatgtgcca ttcctcagat actacgcatc 8207
   ttgttaatat gtatgtatta tgtatgttta tttagtgtgc ccaagtatgc aggtattttg 8267
   ttggagtttt caccttccct tgtgggctct ccgcattaaa ctcagctcct cgggctagtg 8327
   agcaatgcct tcactcgatg agccatctcg ctgcccctgc tgccacctcc tccttatttc 8387
   ccagatggga ctacgcactg cactggccta aagctcacca agtcatccag agtggctagc 8447
   cagggagact cagggatatg ctggcctctg cctccacagt gctagaatta caggcataca 8507
   tcactgctgg aagattttta acctgaatcc tgaggataga gcaggcactc taccaatgga 8567
   gggttctttt tgtgtttggt ttggtttcct ctgcataaga tcaggcagtc tgaaatagtg 8627
   tagcctgggc tacataacat cttgtctcaa aaagcctata gaggtaggga ggtcgaggct 8687
   aaagaagagc cttaagccgg ctgtgatagc acacaggata gcctgcacta tatagcaaga 8747
   ccttgtttca aaaacatgga gggaggggta tgttttaagt gctgggctgt gtaacaggca 8807
   ctaagggagc caatgtagac atttgactaa gaaaggatca tcatcaaagc cgggtgggca 8867
   gggtagaggt tggactacag tggtcaagac ccccatagga agccagtttc ccttcttcct 8927
   ctgggcctca agcctggctc gacggccact gctctcacat gccttctcct ctaggctcgt 8987
   ccaccatg 8995
<210> 2
   <211> 23
   <212> DNA
   <213> Artificial Sequence
<220>
   <223> Description of Artificial Sequence: oligonucleotide
<400> 2
   gagtggtgat gttgagagag cct 23
<210> 3
   <211> 18
   <212> DNA
   <213> Artificial Sequence
<220>
   <223> Description of Artificial Sequence: oligonucleotide
<400> 3
   catagttctc gttccggt 18
<210> 4
   <211> 44
   <212> DNA
   <213> Artificial Sequence
<220>
   <223> Description of Artificial Sequence: oligonucleotide
<400> 4
   gatctcccag gtggtggctg cctcttccag acaggctcgt ccac 44
<210> 5
   <211> 44
   <212> DNA
   <213> Artificial Sequence
<220>
<223> Description of Artificial Sequence: oligonucleotide
<400> 5
   catggtggac gagcctgtct ggaagaggca gccaccacct ggga 44
<210> 6
   <211> 18
   <212> DNA
   <213> Artificial Sequence
<220>
   <223> Description of Artificial Sequence: oligonucleotide
<400> 6
   caacttccta agatctcc 18
<210> 7
   <211> 20
   <212> DNA
   <213> Artificial Sequence
<220>
   <223> Description of Artificial Sequence: oligonucleotide
<400> 7
   attcaggctg cgcaactgtt 20
<210> 8
   <211> 24
   <212> DNA
   <213> Artificial Sequence
<220>
   <223> Description of Artificial Sequence: oligonucleotide
<400> 8
   gcaacagtcg ctggacatca cagg 24
<210> 9
   <211> 20
   <212> DNA
   <213> Artificial Sequence
<220>
   <223> Description of Artificial Sequence: oligonucleotide
<400> 9
   ccacggacaa ctgcgttgat 20
<210> 10
   <211> 20
   <212> DNA
   <213> Artificial Sequence
<220>
   <223> Description of Artificial Sequence: oligonucleotide
<400> 10
   ggctcatagc tactgaactg 20
<210> 11
   <211> 31
   <212> DNA
   <213> Artificial Sequence
<220>
   <223> Description of Artificial Sequence: oligonucleotide
<400> 11
   ccacaacttc ctaagatctc ccaggtggtg g 31
<210> 12
   <211> 31
   <212> DNA
   <213> Artificial Sequence
<220>
   <223> Description of Artificial Sequence: oligonucleotide
<400> 12
   ctgcctcttc cagacaggct cgtccaccat g 31
<210> 13
   <211> 12
   <212> DNA
   <213> Artificial Sequence
<220>
   <223> Description of Artificial Sequence: oligonucleotide
<400> 13
   ctaggcggcc gc 12
<210> 14
   <211> 17
   <212> DNA
   <213> Artificial Sequence
<220>
   <223> Description of Artificial Sequence: oligonucleotide
<400> 14
   catgacgtcg gacttgc 17
<210> 15
   <211> 17
   <212> DNA
   <213> Artificial Sequence
<220>
   <223> Description of Artificial Sequence: oligonucleotide
<400> 15
   ggccgcaagt ccgacgt 17
<210> 16
   <211> 27
   <212> DNA
   <213> Artificial Sequence
<220>
   <223> Description of Artificial Sequence: oligonucleotide
<400> 16
   tgcaaaagta ctgaatataa acttgtg 27
<210> 17
   <211> 30
   <212> DNA
   <213> Artificial Sequence
<220>
   <223> Description of Artificial Sequence: oligonucleotide
<400> 17
   atttgcggcc gctttacata attacacact 30
<210> 18
   <211> 31
   <212> DNA
   <213> Artificial Sequence
<220>
   <223> Description of Artificial Sequence: oligonucleotide
<400> 18
   gggtaccatg gataaagttt taaacagaga g 31
<210> 19
   <211> 30
   <212> DNA
   <213> Artificial Sequence
<220>
   <223> Description of Artificial Sequence: oligonucleotide
<400> 19
   ggaattcggc gccgcagtag caatcaaccc 30
<210> 20
   <211> 12
   <212> DNA
   <213> Artificial Sequence
<220>
   <223> Description of Artificial Sequence: oligonucleotide
<400> 20
   ctaggcggcc gc 12

## Description

The invention relates to regulatory sequences of the mouse villin gene that efficiently drive transgenic expression in immature and differentiated epithelial cells of the intestine and uro-genital tracts.

Villin is a cytoskeletal protein which is mainly produced in epithelial cells that develop a brush-border responsible for absorption as in the digestive apparatus (epithelial cells of the large and small intestine) and in the urogenital tract (epithelial cells of the kidney proximal tubules). Because it is expressed in the proliferative stem cells of the intestinal crypts (16,17), it is believed to be an early marker for commited intestinal cells. The multiple levels of regulation control villin gene activity during mouse embryogenesis (18-20) and account for the strict pattern of tissue-specific expression observed in adults. Moreover, the expression of the villin gene in intestinal epithelial cells is conspicuously maintained in their correspondant carcinomas (21-24).

The specific expression pattern of villin suggests that it is an appropriate candidate for the characterization of regulatory sequences that could allow targeting of heterologous genes into a selected population of cells in the mouse digestive tract.

In order to design new constructs and systems enabling the targeted expression of genes in epithelial cells of intestinal or urogenital tracts, the inventors have investigated the underlying molecular mechanisms and particularly those responsible for the restricted tissue specificity of the expression of villin.

The invention therefore provides new regulatory sequences encompassing cis-acting elements involved in the regulation of the transcription and of the expression of the murine villin gene.

The invention also relates to recombinant constructs comprising said regulatory sequences, for the control of the targeted expression of determined nucleic acid sequences so-called (heterologous sequences or also transgenes), in cells or tissues originating from the intestinal mucosa.

A further object of the invention is to provide cells, tissues or organisms including animals, expressing said determined nucleic acid sequences in a targeted manner.

Transgenic mice are routinely used to study the molecular and cellular basis of normal and pathological states in intestinal mucosa (1-5). The major limitation regarding the targeting of exogenous transgenes in this tissue, is that the epithelium of the mouse intestinal mucosa is renewed every 2-5 days (6-8). The epithelial cells arise from multipotent stem cells functionally anchored at the base (more precisely in the lower third) of the epihelium's proliferative compartment, the crypts of Lieberkühn. These crypts display a monoclonal organization since they are each derived from a single progenitor cell (9). Descendants of stem cells multiply in the middle portion of each crypt (10), and gradually differentiate into four principal cell types. In the small intestine, absorptive enterocytes (constituting >80% of the epithelial cells), mucus-producing globlet cells and enteroendocrine cells migrate upward from the crypts to the apex of surrounding villi (whose colonic counterparts are hexagonal shaped cuffs) (11), where they become apoptotic and are exfoliated into the gut lumen (12). In contrast, antimicrobial peptides-secreting Paneth cells migrate to the bottom of the crypts, where they reside for about 20 days (13).

Given the remarkable protective effect of this epithelium, it is not unexpected that most previous studies aiming to induce neoplastic transformation in intestinal mucosa of transgenic mice have failed (14, 15). In these prior reports, the use of promoter sequences which direct oncogenes in non proliferating enterocytes located in the upper third of crypts produce only minor phenotypic abnormalities without tumorigenic consequences in the gut epithelium, suggesting that the residence time of these villus-associated cells may not be sufficient for the oncogenes to exert their effects. Furthemore this suggests that transgenic mouse models of neoplasia may require an efficient targeting of oncogenes in crypts stem cells or their immediate descendants.

With this goal in mind, the human villin gene has been isolated and characterized (25).

A 2 kb 5'-flanking region has been found to contain sufficient regulatory elements to promote tissue-specific expression of a reporter gene in intestinal and renal cell lines (26). In transgenic mice, this regulatory region is able to drive the expression of the human Ha-ras oncogene in the tissues in which the endogenous gene is actively transcribed. However low levels of expression were observed that did not trigger malignant tissue appearance into the gut of these animals.

The invention provides new means for the targeted expression of heterologous sequences in cells originating from intestinal and/or urogenital tracts. Advantageously, the invention encompasses but is not limited to nucleotide sequences that should overcome at least in part, some of the deficiencies of the previously described gene constructs prepared with regulatory sequences of the human villin gene : especially having recourse to regulatory elements promoting the expression of the murine villin gene, the inventors have designed new DNA constructs that may improve the efficiency of the targeted expression of heterologous genes in transgenic animals, with respect to the results obtained when said heterologous genes are placed under the control of the human villin regulatory elements contained in the above disclosed 2 kb sequence.

The inventors have analysed an extended genomic region of the mouse villin gene with the goal of mapping elements localized at the 5' and/or 3' ends and possibly involved in promoting high levels of targeted expression of heterologous sequences in epithelial stem or differentiated cells, specifically originating from the intestinal mucosa.

As a result, the inventors have identified a transcriptional regulatory region that enables efficient targeted tissue-specific or cell-specific expression, reproducing the expression pattern of the endogenous villin gene in mice, including expression in the crypt stem cells of the colon and in differencitated cells.

In accordance with the invention the identified villin regulatory region provides a molecular tool for the establishment of new cell lines, including new immortalized cell lines, particularly epithelial cell lines originating from intestinal tissue. It also provides means appropriate for the preparation of transgenic organisms, especially transgenic animals, including transgenic mice, enabling the targeted expression of determined nucleotide sequences.

The invention therefore discloses appropriate tools for the study of pathological states related to a dysfunction of gene expression pattern or for the treatment or prevention of said pathological states, for example to enable targeted expression of a gene acting as repairer gene in order to compensate for the dysfunction of an endogenous gene, or to add a novel function in cells or to suppress a determined function or state, or its consequences.

The study of pathological states can benefit from the establishment of transgenic model animals wherein induction of a pathological state is obtained in relation with the targeted expression of genes. It also relates to the study of rescue of pathological states.

The invention thus relates to a nucleotide sequence derived from the 5' sequence of the murine villin gene, having a size of 9 kb on an agarose gel, or a fragment thereof, comprising nucleotide elements having a cis-regulatory activity that promotes the transcription of the murine villin gene.

In accordance with the invention, the expression "nucleotide sequence" designates any type of nucleotide sequence, especially DNA, whatever its origin, including genomic, cloned, amplified, recombinant or synthetic sequences.

The term "derived from" characterizes the fact that the sequence is defined with reference to the isolated endogenous sequence of the murine villin gene. Said nucleotide sequence derived from the murine villin gene nevertheless encompasses sequences obtained without having direct recourse to the native isolated gene. Furthermore, the structural and/or functional characterizing features of the nucleotide sequence may correspond to their native counterpart in the murine villin gene or may be modified as a result of mutation, deletion, truncation, or addition of nucleotides or nucleotide fragments provided its function of promoting the transcription and/or expression of the villin gene is not substantially affected.

The sizes of the nucleotide sequences which are indicated in the present patent application may vary: indeed, the indicated sizes correspond to the size deduced from the band visualized on an agarose gel, in conditions corresponding to those given in the examples.

Especially, a variation of the actual size of within the range of 20% to 5% especially around 15 or 10 % of the sequence, is encompassed within the given size indication.

A nucleotide sequence of the invention is a sequence having a cis-regulatory activity that promotes the transcription of the murine-villin gene thus enabling the transcription ending in the production of mRNA and subsequent expression of villin protein, in intestine epithelial cells. Conditions appropriate to test said regulatory activity are described in the examples.

Where necessary, access to the genomic DNA of the murine villin gene is enabled through various available libraries. Clones obtained from a genomic library can indeed be used to isolate the nucleotide sequences of the invention, provided said clones contain sequences comprising a fragment having a size of at least 9 kb upstream from the translation initiation codon. Said fragment can be obtained from one clone or from overlapping genomic sequences contained in several clones. Probes derived from the cDNA of the villin gene can be used to identify appropriate clones in a genomic library of murine genes. Especially, such probes can be designed aground the translation intiation codon and have advantageously at least 100 nucleotides, including sequences having 200 to 500 nucleotides, or more.

Such probes can also be designed starting from the sequence SEQ ID NO: 1 and can be synthesized or obtained by amplification. They can be derived from the 5' sequence upstream of the translation initiation codon.

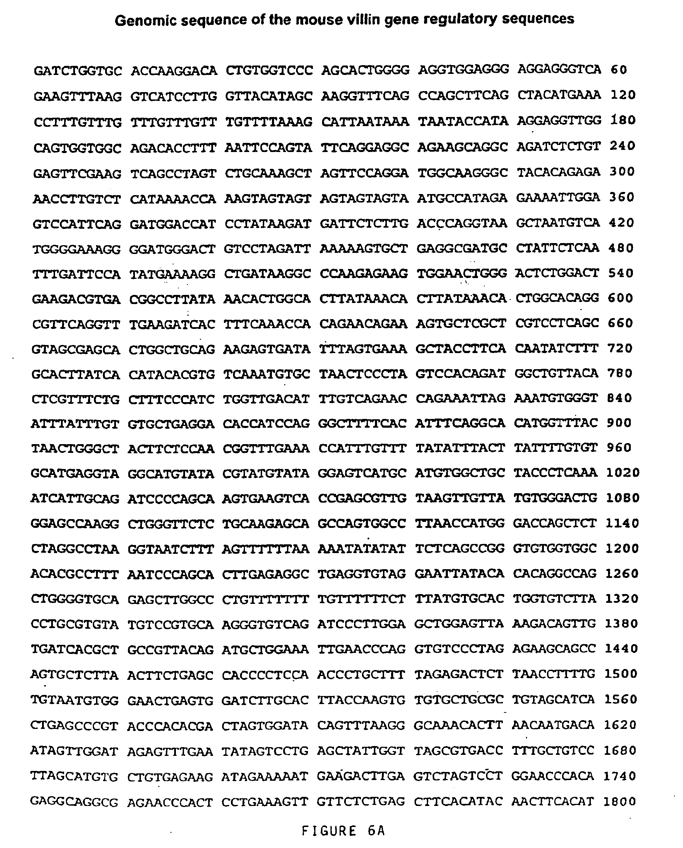

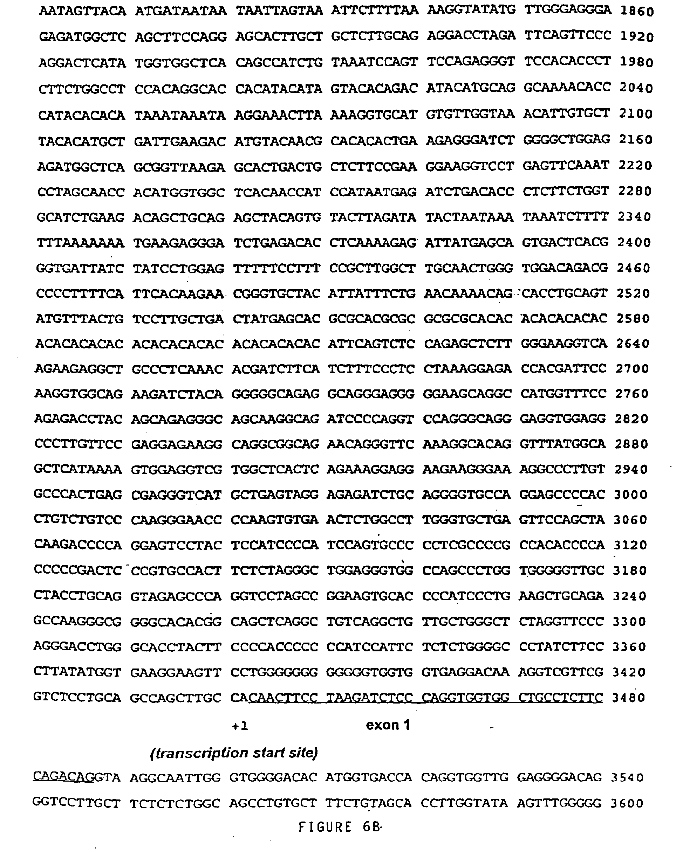

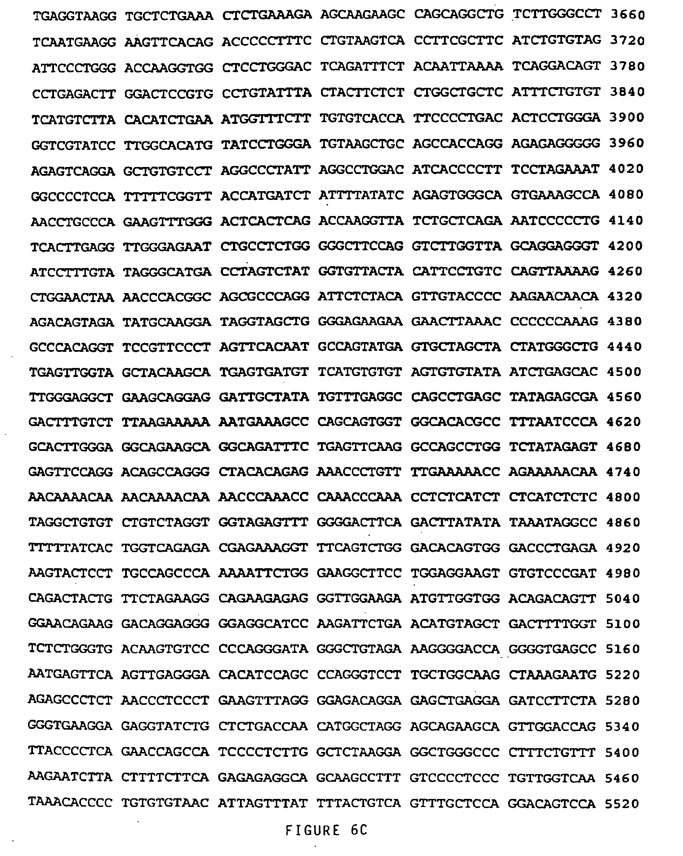

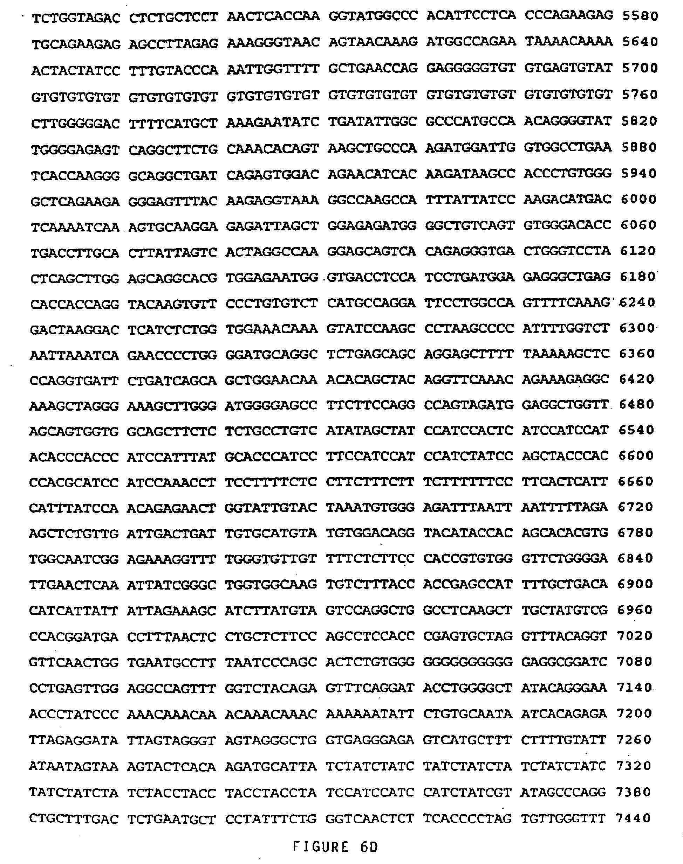

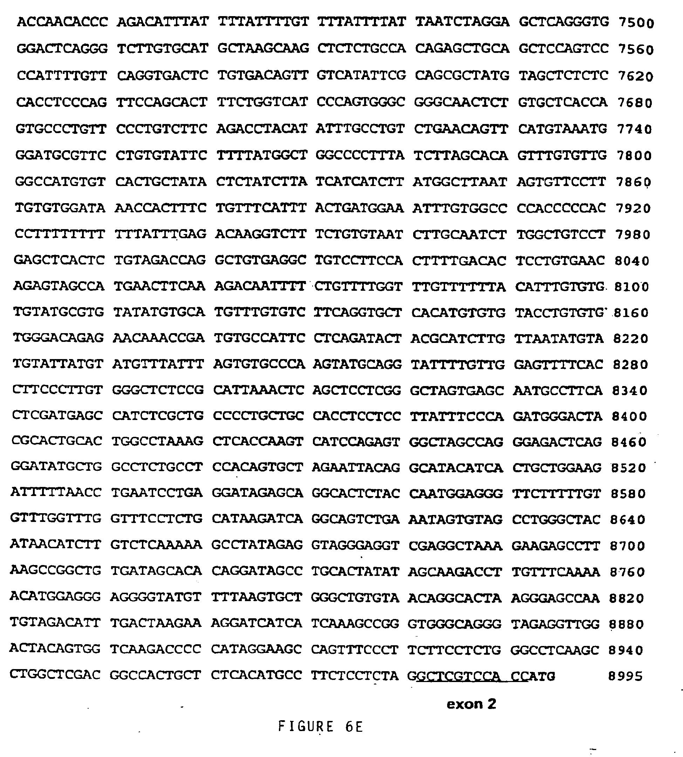

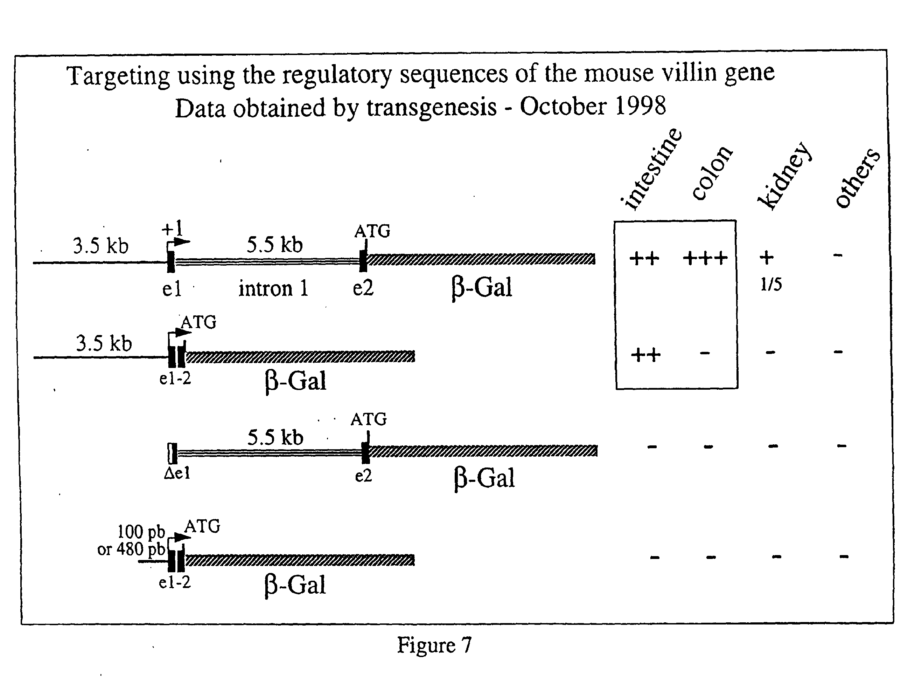

According to specific embodiments, the invention relates to various nucleotide sequences derived from said 9 kb sequence having retained the transcription initiation site of the murine villin gene. Especially, the invention relates to:
- a nucleotide sequence which is the sequence extending 5.5 kb downstream and 3.5 kb upstream from the transcription initiation site of the murine villin gene;
- a nucleotide sequence which is identified as SEQ ID NO: 1 and which is represented on Figure 6;
- a nucleotide sequence which comprises or which is the nucleotide fragment extending from the HS I to the HS IV Dnase 1-hypersensitive sites;

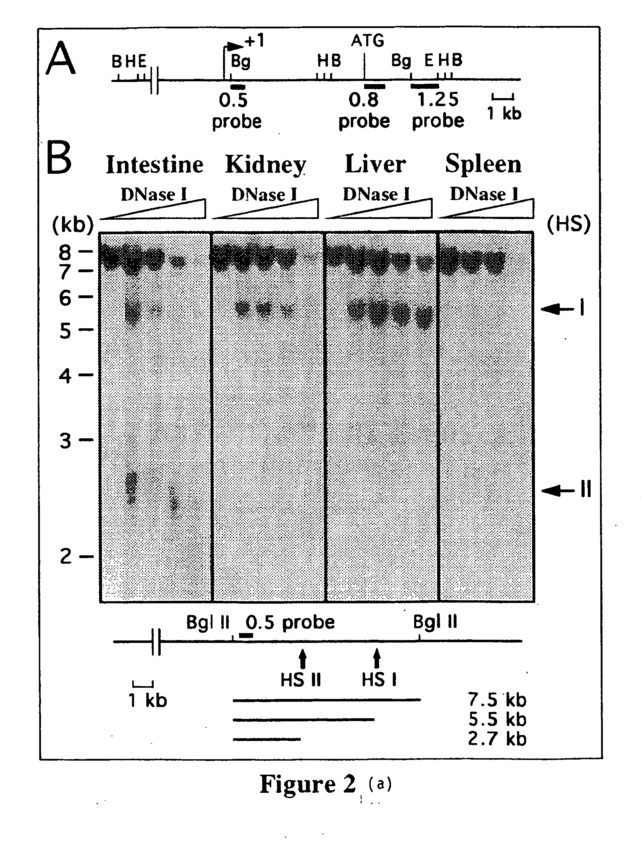

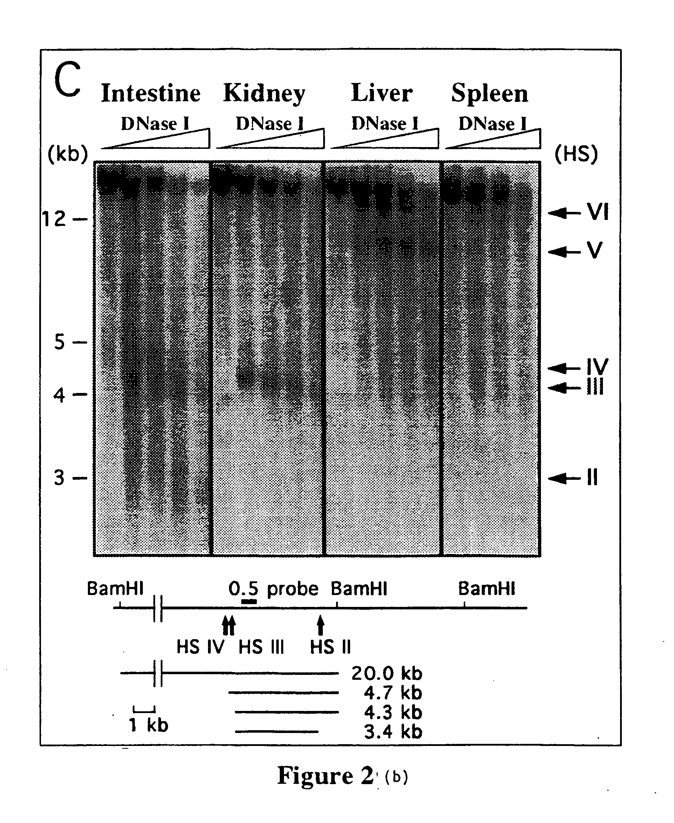

The DNAsel hypersensitive sites can be mapped on a genomic DNA corresponding to the mouse villin gene with the probes described on figure 2.
- a sequence which comprises or which is the nucleotide fragment extending from the HS IV Dnasel-hypersensitive site to the translation initiation codon of the murine villin gene;
- a sequence which comprises or which is the nucleotide fragment extending from the nucleotide at around position -100 upstream from the transcription initiation site, to the translation initiation codon;
- a sequence which comprises or which is the nucleotide fragment extending 3.5 kb upstream from the transcription initiation site to the transcription initiation site and further comprises the translation initiation codon, and possibly the sequence of exon 1 and the sequence of exon 2 starting 5' from the ATG codon;
- a sequence which comprises or which is the nucleotide fragment extending from around the nucleotide at position -480 from the transcription initiation sequence, to the translation initiation codon;
- a sequence which comprises or which is the sequence extending 3.5 kb upstream from the transcription initiation site to the translation initiation codon, provided the region corresponding to intron 1, located between said sites is deleted, or deleted in part;
- a sequence which comprises or which is derived from the nucleotide sequence of the murine villin gene having a size of 9 kb on an agarose gel and extending 3.5 kb upstream from the transcription initiation site and 5.5 kb downstream from said site, or a fragment thereof, said nucleotide sequence or fragment thereof having a regulatory activity on the transcription of the murine villin gene and/or on the level of expression of the murine villin gene in intestine cells and/or in transgenic mice.

The transcription regulatory activity of the murine villin gene is cited as a reference for the identification of nucleotide sequences encompassed within the scope of the invention. The invention furthermore relates to a nucleotide sequence which is derived from said 9 kb sequence of the murine villin gene having retained the transcription initiation site of the murine villin gene and which enables the transcription of a heterologous sequence, with respect to this gene.

Variant nucleotide sequences can be obtained from other animals of the Vertebrates or Invertebrates groups, especially from mammals, or from birds especially chicken or from fishes. Referring to Invertebrates nucleotide sequences can be otained from Drosophila or from *C. elegans.*

Although the above defined sequences have been shown as being able to promote efficient transcription and expression of heterologous sequences in epithelial cells of intestinal origin or in transgenic mice, their structural variations may affect the efficiency of their regulatory activity, either with respect to the tissue - or cell-specificity of this activity, or with respect to the expression level observed.

They can therefore be used to promote the targeted transcription and expression of genes (or more generally any nucleotide sequence of interest) in epithelial cells of the intestinal or uro-genital tracts, either in stem cells or in differentiated cells.

The invention thus relates to a recombinant nucleotide sequence which comprises a first nucleotide sequence as defined in the claims and a second nucleotide sequence for which a tissue specific targeted expression in epithelial intestine cells is sought.

In a particular embodiment of the invention, the second nucleotide sequence is a sequence encoding a determined polypeptide, protein or peptide, all designated hereafter by the term "polypeptide".

The second nucleotide sequence may also be a sequence of therapeutic interest such as tumor suppressor gene, a functional inhibitor of a gene, an antisense sequence an oncogene, an immortilizing gene, a normal gene for the restauration of a function, or more generally any sequence the targeted expression of which in epithelial cells of intestinal origin may present an interest, for instance in processes for preventing, controlling or curing pathological states including those states related to the development of tumors.

In a particular embodiment of the invention, the second nucleotide sequence codes for an antigen or an immunoglobulin or for fragments thereof, including variable chains or immunoglobulins.

In another embodiment of the invention, the second nucleotide sequence is an oncogene.

A recombinant sequence of the invention comprising an oncogene may be used for studies relating to carcinogenesis especially in animal models expressing said recombinant sequence.

According to a specific embodiment of the invention the second nucleotide sequence which is placed under the control of the regulatory sequences of murine villin gene, is further placed under the control of an inducible system, for example the Tetracycline/Doxycycline mediated temporal control of gene expression in transgenic mice (Kistner A. et al, 1996, PNAS, 93, 10933-10938).

The invention also concerns vectors containing the nucleotide sequences of the invention especially plasmids or cosmids, appropriate for the transfer and/or expression of these sequences in cells or transgenic animals.

The invention also concerns recombinant epithelial cells comprising a recombinant sequence as described above.

These epithelial cells encompass stem cells, especially crypt stem cells of the intestinal mucosa, or differenciated cells, especially commited intestinal cells.

The invention further relates to an epithelial cell originating from the kidney proximal tubules recombined with the nucleotide sequences of the invention.

The epithelial cells of the invention can be immortalized cells, especially as a result of the expression of an immortalizing gene (e.g., AgT (tsA58)) the expression of which is drived and targeted by the regulatory nucleotide sequence of the invention.

The administration of the recombinant nucleotide sequence of the invention may be made by the available techniques including ex vivo or in vivo administration processes, especially by electroporation, calcium phosphate precipitation, liposomes...

The invention also concerns transgenic animals obtained by transgenesis of recombinant nucleotide sequences of the invention.

These transgenic animals encompass animals from the Vertebrates or Invertebrates groups and are especially birds for instance chicken or fishes or non-human mammals, more particularly mice. Invertebrates like Drosophila or Nematodes, like *C. elegans* can also be used for the preparation of transgenic animals expressing recombinant sequences of the invention under the control of regulatory sequences derived from their endogenous villin genes. Appropriate processes for the preparation of the transgenic animals expressing in a targeted tissue specific way, recombinant nucleotide sequences, are disclosed in the following examples.

Such a process for the preparation of transgenic mice, advantageously comprises the steps of:
- administration of a transgene into the pronuclei of fertilized eggs of mice,
- enabling the development of the recombined eggs to recover transgenic mice (founders) and verifying the presence of the transgene,
- if appropriate crossing the founders with non transgenic mice.

Further steps, including crossing between mice capable of expressing the sequences of the invention with mice expressing different sequences (e.g. sequences encoding inducible systems) in order to obtain double recombinant mice.

### LEGENDS OF THE FIGURES

**Figure 1**:
   **Determination of the transcription start site of the mouse villin gene by primer extension.** A, primer extension analysis was performed with mouse intestinal total RNA (30 µg) and with either the end-labeled villin oligonucleotide (generating a 105-nuleotides extension product) or the end-labeled mouse intestinal fatty acid binding protein gene (Fabpi) oligonucleotide used as a positive control (generating a 81-nucleotides extension product). The size of the fragments obtained by primer extension is shown at the left. The unrelated sequence ladder that was run in the same gel is used as a size marker. B, nucleotide sequence between the transcription start site (the bold adenosine designated as +1) and the initiation codon (the bold underlined ATG codon) of the mouse villin cDNA. Each of the splice junctions present in the intron 1 (indicated below) conforms to the consensus splice donor (the italique GT nucleotides) and acceptor (the italique AG nucleotides) patterns, described by Breathnach and Chambon (30). C, schematic representation of the organization of the 5' -flanking region of the murine villin gene. The open box represents the untranslated exon and the shadowed box represents the first coding exon. The size of the exon and the intron is indicated.
Figure 2 :
   **DNase I-hypersensitivity in the mouse villin gene.** A, a partial restriction map diagram of the mouse villin gene regions subcloned (-3.5 to +9.9 kb in respect to the transcription start site, indicated by an arrowhead). *Bam*H1 (B), *Bg*/II (Bg), *Eco* RI (E), *Hind* III (H) restriction sites, ATG initiation codon and the probes used to map the hypersensitives sites (0.5, 0.8 and 1.25 kb) are shown. B and C, intestine, kidney, liver and spleen nuclei were digested with increasing amounts of DNase I at 0 °C for 10 min (0, 20, 40, 80, 160 units). 10 µg of purified genomic DNA was digested with *Bg*/II (panel B), and *Bam*HI (panel C), electrophoresed and transferred to a nylon membrane. Hypersensitives sites were revealed by probing with a ³²P-labelled fragment of 0.5 kb. Positions of coelectrophoresed molecular weight markers are indicated at the left, and the hypersensitive bands are marked by arrows at the right. The maps represented below show the position of restriction sites, the deduced DNase I-hypersensitive sites (indicated by arrows) and the 0.5 kb probe used.
Figure 3 :
   **Transient transfection analysis of the mouse villin promoter.** A, above a partial restriction map diagram of the mouse villin gene from 9 kb with respect to the translation initiation codon. *Apa*I (A), *Bam*H1 (B), *Bg*/II (Bg), *Bst*EII (Bs), *Drd*I (D), *Nco*I (N) and *Xba*I (X) restriction sites are shown. The schematic representation below shows the location of the four hypersensitive sites (I-IV) as well as the 5.5 kb intron (represented by a cross-hatched rectangle) separating the transcription start site (indicated by an arrowhead) and the translation initiation codon. B, diagrams of the various constructs generated by deletion. Different portions of the 5'-flanking region of the mouse villin gene were fused with the *E. coli* β-galactosidase gene containing the nuclear localization signal (nls). C, β-galactosidase activities resulting from transient transfections into CaCo2 colon cells (shadowed bars) or LLCPK kidney cells (open bars) with the reporter constructs generated (represented in panel B). Basal activity resulting from the promoterless pBasic plasmid was set arbitrarily at 1.Values indicate the average of at least three independent transfections.
Figure 4 :
   **Expression pattern of the transgene.** Transgene (β-Gal) specific-transcripts were detected by reverse transcription-PCR in a ethidium bromide containing agarose gel. Above each lane, the different tissues tested and the controls, (+) : kidney mRNA from a mouse in which the β-galactosidase was inserted at the villin locus (32), and (-) : distilled H₂O as a template. RT-PCR were also performed on mRNAs of the endogenous villin gene and the ubiquitous TFIID gene.
Figure 5 :
   **β-galactosidase activity in sections of small intestine and colon from transgenic mice.** Tissues were removed from transgenic mice, fixed, and stained for β-galactosidase activity with X-Gal as described. β-galactosidase activity was observed in the epithelial cells, both immature and differentiated, along the crypt-villus axis in the small intestine (panel A). Note that the differentiated cells exhibited a strong signal as did the villus-associated cells and the Paneth cells (arrows) localized to the bottom of the crypt (panel B). The epithelial cells of the colon were also stained (panel C), particularly all the cells in the crypt (panel D). Bars, 100 µm (panels A and C), 40 µm (panels B and D).
Figure 6 :
   Sequence of the genomic DNA of the murine villin gene comprising cis-acting elements capable to promote the transcription of the murine villin gene in intestinal mucosa and kidney proximal tubules. The sequence comprises the transcription initiation site at position 3442 followed by the sequence of exon 1 containing 46 pb, the translation initiation codon at position 8993, the sequence of intron 1 extending from nucleotide 3488 to nucleotide 8981.
Figure 7 :
   Targeted expression, of the β-galactosidase protein using regulatory sequences of the mouse villin gene. The data have been obtained by transgenesis.
Figure 8 :
   Protocols for the preparation of transgenic mice expressing determined nucleotide sequences under the control of the murine villin gene regulatory sequences.
Figure 9 :
   Transgene expression in different Founders, of the large T antigen and K-Ras antigen.

### EXPERIMENTAL PROCEDURES

### I Transcription of the LacZ reporter gene with regulatory regions contained in the 9kb regulatory sequences from the mouse villin gene

A large genomic region of the mouse villin gene has been analyzed. A 9 kb regulatory region of the mouse villin gene (harbouring 3.5 kb upstream the transcription start site and 5.5 kb of the first intron) was able to promote transcription of the Lac Z reporter gene in small and large intestines of transgenic mice, in a transmissible manner, and thus efficiently directed subsequent β-gatactosidase expression in epithelial cells along the entire crypt-villus axis. In the kidney, the transgene was also expressed in the epithelial cells of the proximal tubules but is likely sensitive to the site of integration. A construct lacking the first intron restricted β-galactosidase expression to the small intestine. Thus, the 9 kb genomic region contains the necessary cis-acting elements to recapitulate the tissue-specific expression pattern of the endogenous villin gene. Hence, these regulatory sequences can be used to target heterologous genes in immature and differentiated epithelial cells of the small and/or large intestinal mucosa.

Here we report the analysis of tissue-specific expression of the mouse villin gene using : (i) DNase 1-hypersensitive sites assays, (ii) transient-transfection assays and (iii) transgenic mice. -

### Cell culture and ex vivo transient transfection.

Human colon carcinoma CaCo2 cells were cultured at 37°C, 10% in CO2, in Dulbecco modified Eagle medium supplemented with 10% fetal serum, 1X nonessential aminoacids and 5 mM L-glutamine. Pig Kidney proximal tubules derived-LLCPKI cells and canine kidney distal tubules derived-MDCK cells were cultured at 37°C, 10% CO2, in Dulbecco modified Eagle medium supplemented with 10% fetal calf serum and 5 mM L-glutamine. Cells cultures, approximately 50% confluent in 60 mm-dishes containing serum-free medium, wre cotransfected using 15 µl of Lipofectin reagent (Life Technologies, Inc.) with 5 µg of each β-galactosidase reporter plasmid construct and 5 µg of the control plasmid, pRSVLuc, which contains the luciferase gene under the control of the Rous sarcoma virus promoter. The serum-free medium was changed to growth culture medium 6 h after transfection, and cells were harvested 48 h later. Cell extracts were assayed by chemiluminescent detection of both β-galactosidase (Galacto-Light, Tropix, Inc.) and luciferase (Luciferase Assay Kit, Tropix, Inc.) activities using a luminometer (Berthold). β-galactosidase activity (light units) was corrected for variations in transfection efficiencies as determined by luciferase activity. The volume of cell extracts used in the β-galactosidase and luciferase assays wre adjusted such taht the enzyme activity was always within the linear range of the assay. All transfections were repeated at least three times. Results are expressed as -fold induction over that of the vector without promoter, pBasic.

### Primer extension analysis.

Total RNA was isolated from mouse intestine with RNA NOW reagent (Biogentex) under the conditions suggested by the supplier. For primer extension assay, 2 ng of ³²P-labeled oligonucleotide probe (5'-GAGTGGTGATGTTGAGAGAGCCT-3') complementary to nucleotides +81 to +103 of the murine villin cDNA (GenBank Accession No. M98454) was hybridized with 30 µg of total RNA at 60°C (0.25 M KCI, 10 mM Tris-HCl, pH 7.5, 1 mM EDTA) for 90 min. Transcription with 5 U/µl of Moloney murine leukemia virus reverse transcriptase (Life Technologies, Inc.) was carried out at 37°C for 90 min in a 300 µl of a solution containing 75 mM KCI, 3 mM MgCl₂, 50 mM Tris-HCl (pH 8.3), 10 mM dithiothreitol, 0.75 mM deoxynucleoside triphosophates, 75 µg/ml actinomycin D and 0.3 U/µl RNasin. The primer extension products were separated by electrophoresis in denaturing 8% polyacrylamide gels. The full-length extension product (105 nucleotides) was obtained by comparison with the length of the comigrating sequencing reaction products. A primer extension control experiment was performed on the same total RNA preparation, using a ³²P-labelled oligonucleotide probe (5'-CATAGTTCTCGTTCCGGT-3') complementary to nucleotides +63 to +80 of the mouse intestinal fatty acid binding protein (I-FABP) cDNA and generating a 81-nucleotide extension product (27).

### DNase 1-hypersensitive sites analysis.

Tissues from 30 mice were used per assay of intestine, kidney, liver and spleen. Nuclei preparation and DNase I digestion were performed as described (28) with minor modifications. Nuclei were digested without or with 20 to 160 units of DNase I (DPRF Worthington) for 10 min at 0°C. Genomic DNA was purified by three rounds of (1:1) phenol-chloroform extraction followed by chloroform extraction and precipitation with ethanol. 10 µg of each sample was digested overnight with restriction enzyme (*Bam*HI or *Bgl*II). The DNA fragments were separated by electrophoresis on a 0.8% agarose gel in TAE (40 mM Tris (pH 7.2), 20 mM sodium acetate, 1 mM EDTA), transferred onto a charged nylon membrane (Hybond-N⁺, Amersham), and hybridized at 65°C overnight with a random-primed (rediprime, Amersham) ³²P-labeled probe. The probe, the *Bgl*II-*Pst*I probe (0.5 kb) (as indicated in Fig. 2) was used to map the DNase 1-hypersensitive sites in the BamHI, BglII fragments. The filter was washed using (1X SSC (0.15 M NaCL, 15mM sodium citrate, pH 7.0), 0.1 % SDS, 55°C) and exposed to film overnight at -70°C with an intensifying screen.

### Plasmids construction.

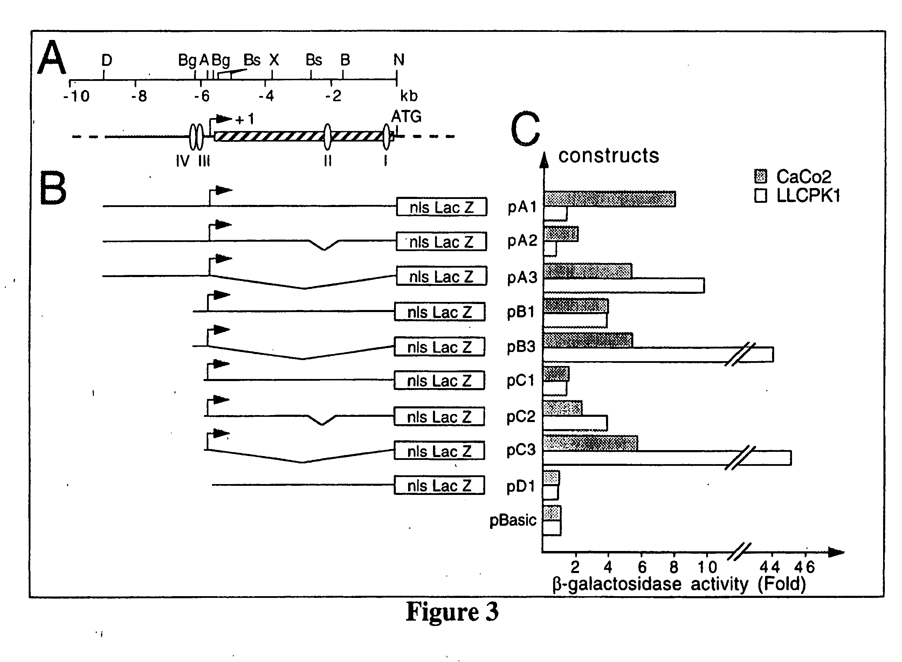

All constructs described were subcloned into the pBluescript II KS vector (Stratagene) with fragments isolated from a λDASHII phage containing a 16.3 kb region (9 kb upstream and 7.3 kb downstream from the translation initiation codon) of the mouse villin gene (29). The pD1 construct (as described in the Fig. 3B) was prepared by ligating a *Bam*HI fragment of 5.1 kb (1.8 kb upstream from the ATG translation initiation codon of the mouse villin gene, subcloned 5' to the nuclear localization signal-galactosidase gene-SV40 polyadenylation site, using a polymerase chain reaction (PCR) strategy) at the *Bam*HI site in a plasmid containing the 3.7 kb region of the mouse villin gene (immediately 5' to the 1.8 kb region described above). The pA1 and pA2 (containing an internal 1 kb deletion) constructs have resulted from several steps based on the *Bst*EII sites present in the 3.7 kb region described above and in a plasmid containing the 3.5 kb region of the mouse villin gene (immediately 5' to the 3.7 kb region). The pC1 and pC2 constructs were derived from the pA1 and pA2 plasmids cut with *Apa*I and re-ligated, respectively. To generate the pB1 construct, a *Bgl*II fragment (480 bp) from the 3.5 kb region described above was excised and cloned into the *Kpn*I site of the pC 1 plasmid. The pA3, pB3 and pC3 constructs correspond to the pA1, pB1 and pC1 deleted from the intron 1 (Fig. 3B). The sequence between the transcription initiation start site and the translation initiation codon, excluding the intron 1, was deduced from that of the murine villin cDNA (GenBank Accession No. M98454) and was introduced into the *Bgl*II-*Nco*I sites of the pC1 construct by using a dimerized oligodimer made of a coding-strand oligonucleotide (5'-GATCTCCCAGGTGGTGGCTGCCTCTTCCAGACAGGCTCGTCCAC-3') and a non coding-strand oligonucleotide (5'-CATGGTGGACGAGCCTGTCTGGAAGAGGCAGCCACCACCTGGGA-3'), resulting in the pB3 construct. The pA3 and the pC3 constructs were derived from the pB3 plasmid by ligating an ApaI fragment (3.1 kb) and a *Bg*/II fragment (480 bp) from the 3.5 kb region described above, at the *Apa*I site in the pB3 plasmid respectively. Subcloning steps were confirmed by DNA sequencing.

### Transgenic mice generation.

The transgenes digested with *Xho*I-*Not*I, purified by gel elution and Elutip Columns (Schleiche & Schuell). The linear fragments were supended in 10 mM Tris-HCl, pH 7.4, 0.2 mM EDTA and were injected into the pronuclei of the fertilized eggs of the B6/D2 mice. Mice carrying transgenes (founders) were first identified by PCR of genomic DNA isolated from a short segment of tail to confirm the presence of the galactosidase gene and then analyzed by Southern blotting to determine the copy number of the integrated transgene. Each founder animal harbored one copy of the transgene per genome. Small intestine, colon, kidney, stomach, liver, heart, lung, thymus, brain, spleen and muscle were dissected from transgenic mice, cut in small pieces, quickly frozen in liquid nitrogen-cooled isopentane either prepared for total RNA extraction or embedded in Tissue-Tek O.C.T. Compound (Sakura Finetek) blocks to perform cryosections.

### Reverse transcription-PCR Analysis.

Total RNA was isolated from mouse tissues described above, with SV Total RNA Isolation System (Promega) under the conditions suggested by the supplier. 20 ng of pd(N)₆ random primer (Pharmacia) were hybridized with 2 µg of total RNA at 70°C for 10 min in distilled water. Reverse transcription with 200 U of Moloney murine leukemia virus reverse transcriptase (SuperScript II, Life Technologies, Inc.) was carried out at 37°C for 90 min in a 20 µl solution of 1X First Strand Buffer (Life Technologies, Inc), 10 mM dithiothreitol, 0.5 mM deoxynucleoside triphosphates and 0.4 U/µl RNasin. 2 µl of the resulting cDNAs, were amplified by PCR reaction in 50 µl for 40 cycles. Each cycle consisted of 60 sec at 94°C, 60 sec at 51°C (for transgene and villin) and 57°C (for TFIID), and 30 sec at 72°C. For the transgene primers, 5'-CAACTTCCTAAGATCTCC-3' coding strand and 5'-ATTCAGGCTGCGCAACTGTT-3' non-coding strand were used, generating a 250 bp product. For villin amplification 5'-CAACTTCCTAAGATCTCC-3' coding strand primer and 5'-GCAACAGTCGCTGGACATCACAGG-3' non-coding strand primers were used, generating a 473 bp product; for TFIID amplification 5'-CCACGGACAACTGCGTTGAT-3' coding strand primer and 5'-GGCTCATAGCTACTGAACTG-3' non-coding strand primer were used, generating a 220 bp product. In all cases, one-fifth of the PCR product was run on an ethidium bromide containing agarose gel.

### Detection of β-galactosidase activity.

Cryosections (5 µm) from the tissues described above wre then dried overnight at room temperature, fixed with 3% paraformaldehyde for 5 min, washed in phosphate buffered saline and incubated in a staining solution that contained 0.4 mg of 5-bromo-4-chloro-3-indolyl-β-D-galactopyranoside (X-Gal) per ml, 4 mM potassium ferricyanide, 4 mM potassium ferrocyanide, 2 mM MgCl, at 37°C for 8 h. After staining, the sections were again washed in phosphate buffered saline, mounted and examined histologically to detect the expression of exogenous β-galactosidase.

### RESULTS

### Determination of the transcription start site.

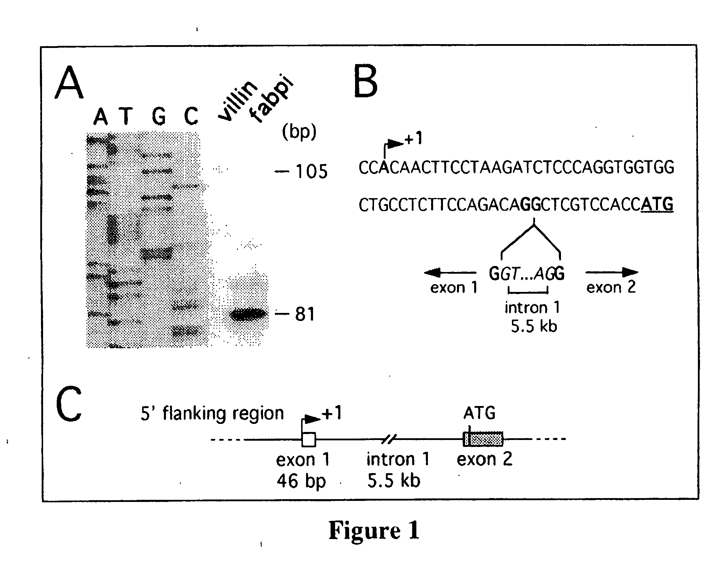

To determine the transcriptional start site of the mouse villin gene, total RNA was isolated from intestine and analysed by primer extension assay using an oligonucleotide complementary to the mouse villin cDNA downstream of the ATG translational start site. The efficiency of the reaction was confirmed by primer extension of the mouse intestinal fatty acid binding protein (I-FABP) gene (fabpi) from the same RNA preparation (27). Analysis of the fabpi extension product on a sequencing gel by comparison with a sequence ladder (Fig. 1A) revealed a strong signal band of a size of 81 bp as expected. The extension product of villin was 105 pb indicating that the transcriptional start site (an adenosine residue subsequently designed as nucleotide +1) was 57 nucleotides upstream of the translation initiation codon of the murine villin cDNA (Fig. 1B). Comparison of the genomic sequence encompassing 9 kb upstream from the ATG initiation codon with the cDNA sequence, position of splice site consensus sequences in the 9 kb genomic sequence (Fig. 1B) and determination of the transcription start site reveal that the mouse villin gene has one transcription start site which is separated from the ATG iniation codon by a 5.5 kb intronic region (Fig. 1C).

### DNase I-hypersensitive sites in the mouse villin gene.

To characterize the key regulatory regions involved in the specific control of villin expression, we have mapped the DNase I-hypersensitive sites (31) in the mouse villin gene (along a region extending 9 kb upstream and 4.4 kb downstream from the translation initiation codon, as represented in Fig. 2A). The chromatin form of the mouse villin gene in different tissues (intestine, kidney, liver and spleen) was submitted to limited DNase I digestion and subsequently digested with the appropriate restriction enzymes. Accordingly, nuclei were isolated from intestine, kidney, liver and spleen. The Dnase I-digested DNA was restricted by BlgII and hybridized with a 0.5 kb probe homologous to the 5' of the 7.5 kb BlgII fragment (Fig. 2B). Two sets of Dnase I incubation-related fragments were detected, migrating at 5.5 and 2,7 kb, and corresponding to hypersensitive sites designated as HS I (located at approximately +5.5 kb downstream from the transcription start (+1) site, just upstream the ATG initiation codon) and HS II (located at approximately +3 kb downstream from the (+1) site), respectively. HS I was observed in nuclei isolated from intestine, kidney and liver, whereas HS II was only present in intestinal tissue. No specific hypersensitive sites were detected in nucleic isolated from spleen. The presence and location of these hypersensitives sites were detected in nuclei isolated from spleen. The presence and location of these hypersensitives bands were confirmed by stripping and rehybridizing the same blot with the 0.8 kb probe (Fig. 2A) homologous to the other end of the 7.5 kb BglII fragment (data not shown). Using BamHI digestion and the 0.5 kb probe (Fig. 2C), five sets of DNase I-treated nuclei-related fragments were detected, migrating at 3.4, 4.3, 4.7 and approximately 10 and 15 kb, corresponding to the hypersensitive sites HS II (previously identified and consequently confirmed), HS III (located at approximately -0.5 kb upstream from the (+1) site), HS IV (located at approximately -1 kb upstream the (+1) site), HS V (located at approximately -10 kb upstream from the (+1) site) and HS VI (located at approximately -15 kb upstream from the (+1) site), respectively. HS III was observed in nuclei isolated from both intestine and kidney, whereas HS IV was only present in intestinal tissue as HS II. The hypersensitive site HS V and HS VI were only present in liver tissue (in which villin is weakly expressed) and were located far upstream from the transcription start site in regions (i) which have not been subcloned and (ii) which could-belong to an adjacent gene; for these reasons, these hepatic-specific hypersensitives sites were not analyzed further. As for BglII digestion, no specific hypersensitive sites were detected in nuclei isolated from spleen. Using other independent restriction digestions (EcoRI and Hindus) and the 0.5, 0.8 and 1.25 kb probes (Fig. 2A) to map the locations of the hypersensitive sites, similar results were obtained (data not shown).

In conclusion, four major distinct DNase I-hypersensitives sites (HS I to HS IV) were shown to be present in the region extending from -1 kb to +5.5 kb in respect to the transcription start site (Fig. 3A) of the mouse villin gene. These sites were detected in intestine (HS I to HS IV), kidney (HS I and HS III) and liver (HS I), tissues in which villin is expressed, but they were not found in spleen, a tissue that does not produce villin. These findings correlate with the tissue-specific control of villin gene expression, and suggest that the putative critical regulatory elements lie within these regions. HS II and HS IV were only detected in intestine and are probably associated with tissue-specific transcription factors binding sites involved in the positive control of villin gene intestinal expression.

### Analysis of promoter activity by transient expression.

To test the effects of the segments containing the DNase I-hypersensitives sites (Fig. 3A) on transcriptional activity and to define more precisely the element(s) controlling villin gene expression in the intestine, segments were subcloned upstream of a promoterless Lac Z plasmid (coding for the bacterial β-galactosidase gene with a nuclear localization signal sequence) (Fig. 3B). The resulting recombinant plasmids were tested by transient transfection assays in cultures cell lines. The construct pA1 contained all the subcloned regions dowstream from the ATG initiation codon, encompassing the four DNase I-hypersensitive sites (HS I to HS IV) described above and the 5.5 kb intronic sequence, intron 1. Plasmids pA2 and- pA3 were identical to pA1 except for the presence of intestine-specific hypersensitive site HS II and intron 1, respectively. Plasmid pB1 and plasmid pC1 were similar to plasmid pA1, but lacked the regions extending from -480 bp to -3.5kb and -100 bp to -3.5 kb according to the transcription start site, respectively. Plasmid pC2 was identical to pA2, but lacked the region extending from -100 bp to -3.5 kb. Plasmids pB3 and pC3 were identical to pB1 and pC1 except for the presence of intron 1, respectively. The plasmid pD1 was identical to pA1 except for the presence of the transcription start site and the region extending upstream from this site. The plasmid pBasic, which does not contain a promoter or enhancer, and a pControl plasmid which possesses the SV40 promoter, were also tested in each experiment. Transient transfections were performed in the human colon enterocyte-like CaCo2 cell line and the pig kidney proximal tubules-derived LLCPK1 cell line which express villin, and in kidney epithelial cells in which no villin expression is detected, MDCK (a canine kidney distal tubules-derived cell line). Transcription from the villin promoter was measured by assaying β-galactosidase activity in extracts made from the transfected cells, and the results were expressed as - fold induction over that of the promoterless vector, pBasic (Fig. 3C). High levels of β-galactosidase activity in the pControl transfected cell lines (CaCo2 cells, 50-fold over that of pBasic; LLCPK1 cells, 98-fold) demonstrated the presence of efficient general transcription/translation machineries in these cells. Very low levels of β-galactosidase activity in pD 1 both transfected cells compared to pBasic transfected cells showed that the transcription start site was necessary for an efficient specific transcription of the reporter gene and that nonspecific transcription was not initiated elsewhere in the villin regulatory sequences. The construct pA 1 expressed the β-galactosidase gene at the highest level in CaCo2 cells (8-fold over pBasic) as compared with LLCPK1 cells (1.5-fold over pBasic) suggesting that the four DNase I-hypersensitives sites together with the first intron are necessary to promote efficiently transcription in cells of intestinal origin. Deletion of the fragment containing the intestinal-specific hypersensitive site HS II (pA2) dramatically decreased β-galactosidase expression in CaCo2 cells (2-fold over pBasic) to about 25% of that of pA1, demonstrating that a major element which confers intestinal activity was confined within this fragment. Similar results were obtained when the region upstream from the transcription start site (encompassing HS III and HS IV) was almost wholly deleted with or without HS II (pC1 and pC2, respectively). The deletion of the intronic region alone (pA3), or in combination with deleted sequences upstream from the transcription start site (pB3 and pC3 extends only from -480 and - 100 bp, respectively), affected to a lesser extent β-galactosidase expression in the same intestinal cells (5.5-fold over pBasic), with a decrease to only about 65% of that pA1, demonstrating that the regulatory elements which lay within 100 bp were sufficient to promote transcription in cultured cells. However the level of β-galactosidase activity increased strongly when the plasmids pA3, pB3 and pC3 were transfected in LLCPK1 cells (10, 44, and 45-fold over pBasic, respectively) showing that the absence of the first intron, in combination with the lack of intestine-specific HS IV, was able to promote transcription in a kidney cell line. This would suggest that negative elements which confer repression in kidney transcription are confined in these elements.

To test specificity, the villin promoter-related constructs were transfected in MDCK cells, which do not express villin. After transfection, these cells showed only base-line levels of β-galactosidase activity when compared to pBasic-related activity (data not shown), demonstrating that the villin regulatory sequences were unable to promote efficient transcription in nonexpressing villin cells, and that consequently the expression of the reporter gene in CaCo2 and LLCPK1 cells is specifically dependent upon these regulatory sequences. Taken together, these results from transient transfection of cultured cells demonstrate that (i) the mouse villin genomic sequence, extending from - 3.5 to +5.5 kb, directs specifically an efficient level expression of the β-galactosidase reporter gene in intestine-derived cells, (ii) this level is dramaticaly reduced when the intronic intestine-specific hypersensitive site HS II or the region upstream from the (+1) site is deleted, (iii) lack of the entire first intron seems to partially restore the intestine-related ability in promoting transcription, and (iv) lack of the entire first intron in combination with intestine-specific hypersensitive site HS IV is correlated with a strong increase of ability in promoting transcription in kidney-derived cells.

### Analysis of transgenes expression in mice :

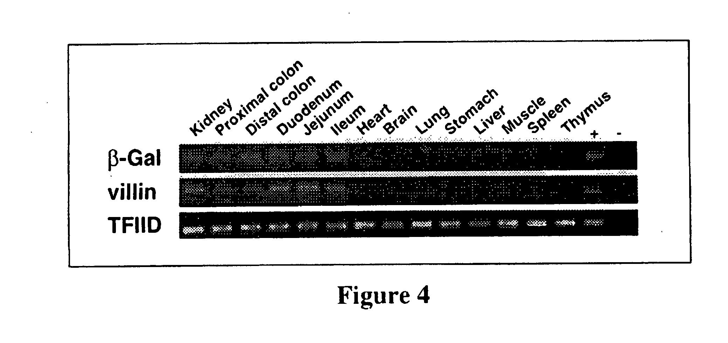

Since the -3.5 to +5.5 kb region of the mouse villin contained the enterocyte-like-specific promoter/enhancer activity in transient-transfection assays, we examined the ability of this region to drive intestine-specific expression of the β-galactosidase reporter gene in transgenic mice. The construct pA1 was then prepare, after excision of the plasmid sequence, and injected into fertilized eggs. Five founder animals which contained the pA1 construct as a transgene- were obtained. The founder mice were analysed for mRNA reporter gene expression in several adult tissues by reverse-transcription PCR (RT-PCR) analysis. From the same cDNA samples, products encoding β-galactosidase, villin and TFIID were analyzed. The PCR assays enabled only the detection of spliced transcribed mRNA, excluding that from genomic DNA itself, by means of an exon-connection strategy by combination of a 5' PCR primer from within the mouse villin promoter sequence just upstream of the splice donor site, and the 3' primers from within the β-galactosidase gene or the villin gene. For each founder, no reporter gene expression was detected in the tissues in which villin mRNAs were not detected using the PCR assay (Fig. 4). For all founder mice, the reporter gene transcription was detected along the cephalocaudal axis of the gut (duodenum, jejunum, ileum, proximal and distal colon) following the intestine-specific expression of the villin gene (Fig. 4). In the kidney, the transgene was only transcribed in one founder of five animals obtained (Fig. 4). TFIID mRNA was present in all samples from tissues in which the reporter gene expression could not be detected (Fig. 4), confirming the quality of RNA from these tissues.

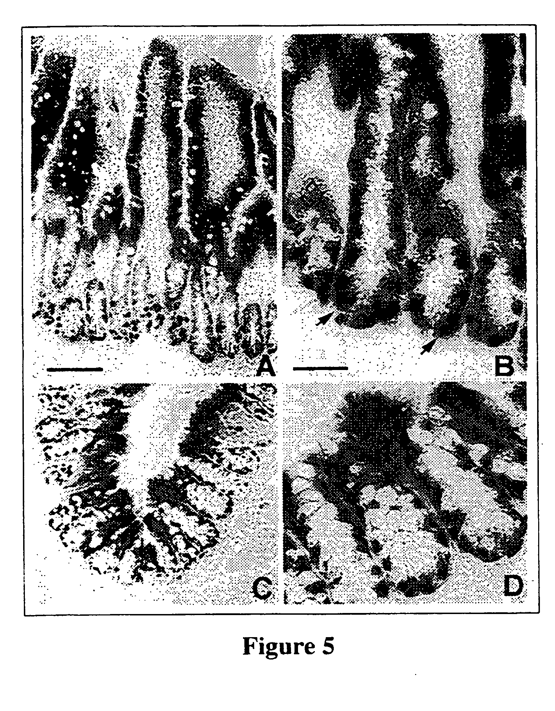

To examine the precise cellular distribution of transgene expression within the tissues, cryostat sections of small intestine, colon and kidney were prepared and subsequently stained for β-galactosidase enzyme activity. Immunofluorescence analysis of β-galactosidase expression was also performed on the same sections and similar results were obtained with the two procedures. Sections of small intestine, colon and kidney from non-transgenic animals exhibited no detectable β-galactosidase activity. For four of five transgenic mice, a heterologous pattern of expression in small intestine and colon was observed in this assay. This heterogeneity was due to mosaicism since we examined founder animals. The expression was confined to the nucleus of the epithelial cells, as expected because the β-galactosidase gene contains a nuclear localization sequence signal (Fig. 5). The staining was detected by a stronger signal in the villi migrating cells when compared with the crypts cells, of both small intestine (Fig. 5A) and colon (Fig. 5C) epithelium, thus confirming that the -3.5 to +5.5 kb region of the mouse villin gene is able to recapitulate precisely the cellular pattern of expression, along the crypt-villus differentiation axis, of the endogenous villin gene (17). A continuous labelling of all cells of the crypt (Fig. 5B and D) was observed, suggesting the expression of the transgene in the stem cells (10). It is noted worthy that the intensity of the β-galactosidase staining was similar to that of intestinal sections from chimeric animals 0which possess a β-galactosidase gene integrated at the villin locus by homologous recombination procedure (32), indicating that the -3.5 to +5.5 kb region of the mouse villin gene was able to promote intestinal transcription as efficiently as the mouse villin gene itself. In the kidney of the founder mouse in which the transgene was detected by RT-PCR, the staining was only observed in the epithelial cells of the proximal tubules where the villin gene is expressed. The founder animals were able to transmit the transgene to their offspring with a similar pattern of β-galactosidase expression. In our attempt to direct an efficient expression of the reporter gene in the intestinal epithelium with shorter regulatory sequences, plasmids pA3, pB3 and pC3 were used to generate transgenic mice, because these constructs display efficient levels of β-galactosidase activity in intestine-derived CaCo2 cells. The presence of the transgene assessed by β-galactosidase staining and immunofluorescence procedures was observed in three of the four independent lines of pA3 transgenic mice generated. These three lines expressed the reporter gene only in the small intestine (in both the immature and differentiated epithelial cells along the crypt/villus axis), and all three lines failed to express the transgene in the other tissues tested, particularly note worthy is the lack of expression in the colon and the kidney (data not shown). These results demonstrate that (i) the 3.5 kb regulatory region upstream the transcription start site of the mouse villin gene is necessary and sufficient to sustain expression strictly in small intestine of transgenic mice, (ii) the first intron of the mouse villin gene is required for colon and kidney expression in transgenic mice. Concerning the pB3 and pC3 transgenic mice, no transgene expression was observed in all tissues examined, including small intestine, colon and kidney. Thus, the key cis-acting elements of the villin gene required for intestinal and/or kidney-related expression of transgene(s) in transgenic mice are not located only within the region encompassing -480 bp upstream from the transcription start site, as observed in the cultured epithelial cells.

### DISCUSSION

In this report, we demonstrate that cis-acting sequences located within a 9kb region (-3.5 to +5.5 kb from the start site of transcription) of the mouse villin gene are sufficient to direct both correct tissue-specific and high expression level of the β-galactosidase reporter gene in transgenic mice, when compared with the endogenous gene (19). Reporter gene expression is detected in the whole intestinal tube and appropriately restricted to epithelial cells along the crypt-villus axis of both small intestine and colon. In addition, these regulatory elements can maintain a gradient of β-galactosidase gene expression from the crypts of Lieberkühn to tips of villi that precisely reproduce the gradient exhibited by the murine villin gene (17). Similarities between transgene and endogenous gene expression were also noticed as judged by a comparison with the staining intensity of β-galactosidase activity in intestinal sections from our transgenic mice and mice in which the reporter gene has been inserted at the natural villin locus by homologous recombination (32).

In the kidney, for only one animal of five analyzed, mouse reporter gene expression was restricted to epithelial cells of the proximal tubules recapitulating the villin expression pattern in this tissue. This suggests that transcriptional mechanisms specifying gene expression to intestine and kidney tissues are in the -3.5 to +5.5 kb region of the mouse villin gene, and that those related to kidney may be sensitive to positional effects. Indeed it is known that the transgene expression is dependent on site of chromosomal integration, and can be influenced by regulatory regions in the vicinity, presumably acting on chromatin conformation (33). The construct lacking entirely the first intron of 5.5 kb, but which harboures 3.5 kb 5' to the start site of transcription of the mouse villin gene, placed in front of the j3-galacosidase gene, restricts the *in vivo* expression of the reporter gene only into the epithelial cells along the crypt-villus axis of the small intestine. The extinction of the reporter gene expression in the kidney might be due to strong positional effects, as reported above, whereas the extinction related to the colon might be due to the absence of regulatory elements of the intron 1, such as the intestine-specific DNase I-hypersensitive site HS II. Constructs harbouring only the first 480 bp and 100 bp 5' to the start site of transcription, in combination with the lack of the first intron, placed in front of the β-galacosidase gene, both failed to drive intestine-specific and kidney-specific expression of β-galactosidase, suggesting that the intestine-specific DNase I-hypersensitive site HS IV localized just upstream from the 480 bp might play an important role in promoting reporter gene expression into the epithelial cells of the small intestine. Thus, distinct and separable regulatory elements in the mouse villin gene may direct transgene expression along the cephalocaudal axis of the gut: the regulatory elements required for transgene expression in the small intestine might be localized in the 3.5 kb region (i.e. the HS IV site) upstream from the transcription start site, whereas those necessary for the colonic expression might be localized in the first intron (i.e. the HS II site). The inability of shorter regulatory sequences of the mouse villin gene to direct correct expression of the reporter gene in the whole intestine of transgenic mice might also be explained by spatial rearrangement of chromatine structure due to the lack of the entire first intron. In fact, the results described here are reminiscent of those of the adenosine deaminase gene (34) and the aldolase Bgene (35) in which elements located in the first intron could be required for transgene expression *in vivo,* because they may contain cis-acting tissue-specific enhancer elements and/or elements involved in promoting decondensation of the chromatin structure, allowing the accessibiliy for transcription factors and RNA polymerase.

To explain the discrepancy seen in the ability of the mouse villin gene regulatory elements to promote transcription of the reporter gene in cell cultures versus transgenic animals, we may argue that the regulation of gene expression in the intestinal epithelium occurs as cells differentiate and migrate along the crypt-villus axis. This process depends on the contacts that these cells maintain with others neighboring cells on the one hand, and with the extracellular matrix on the other hand (36). Thus, an *ex vivo* system as the intestine-derived CaCo2 cell line used in the study, is limited by its weak ability in recapitulating the temporal and spatial complexities of this epithelium and emphasizes the importance to use *in vivo* models to define a function for specific regulatory sequences (37, 38).

Previous studies carried out in transgenic mice to map transcriptional regulatory elements responsible for intestinal expression have been performed using cis-acting - sequences of genes expressed in villus associated-enterocytes of small intestine (4, 5, 38-40). In some of these cases, precocious activation in the crypts in combination with extended expression in the colon occurs in an inappropriate manner. Thus, to our knowledge, the 9kb regulatory region of the mouse villin gene represents the only characterized cis-acting sequences reported today that allow the expression of a heterologous gene in small intestine and colon epithelial cells of transgenic mice reproducing with great fidelity the tissue-specific and cell-specific pattern of expression when compared with that of the endogenous gene itself. In addition, the mice lines that drive a transgenic expression exclusively restricted to the intestinal mucosa could already be studied after selection of those which will not display expression into the kidney because of the positional effects.

The ability to target genes of interest in transgenic mice following the villin restricted-pattern of expression, and particularly in the crypts stem cells enables to the development of targeted genes in animal models. Experimental mouse models reproducing several steps of human colorectal carcinogenesis (a possible genetic pathway has been proposed by Fearon and Vogelstein (41)) could for instance be obtained by efficiently targeting the associated oncogenes or mutated tumor suppressor genes to colonocytes using the villin regulatory region. Another use could lie in the establishment of new cell lines derived from the digestive tract by targeting a thermosensitive SV40 T antigen to the crypts resident-progenitors of intestinal cells, as used in other systems (42-44).

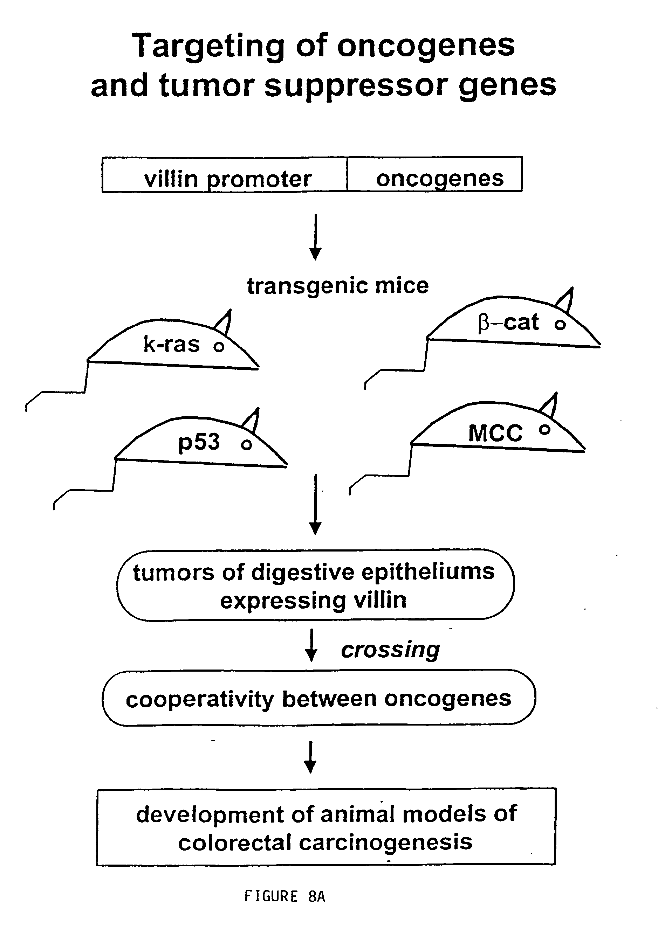

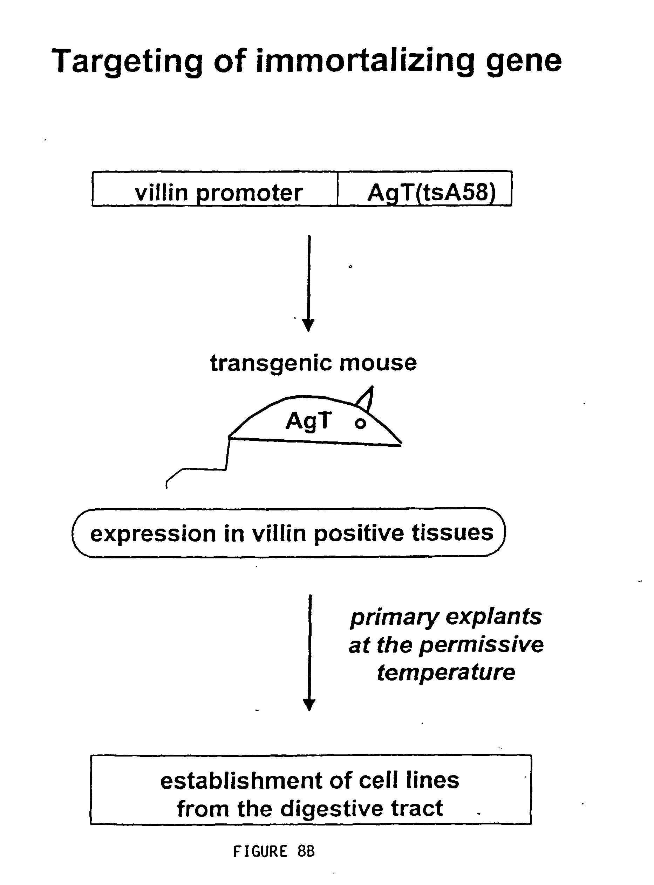

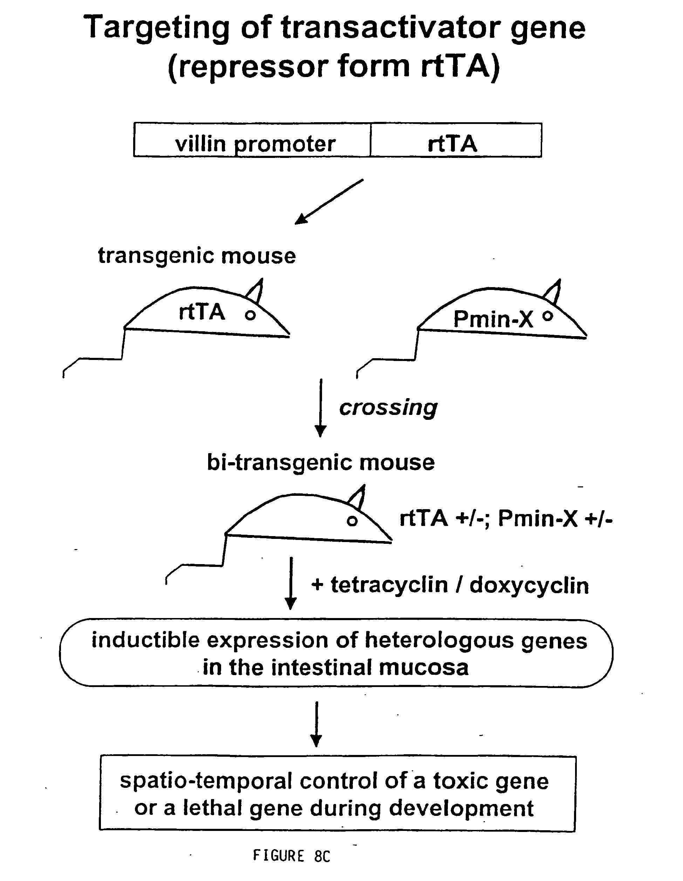

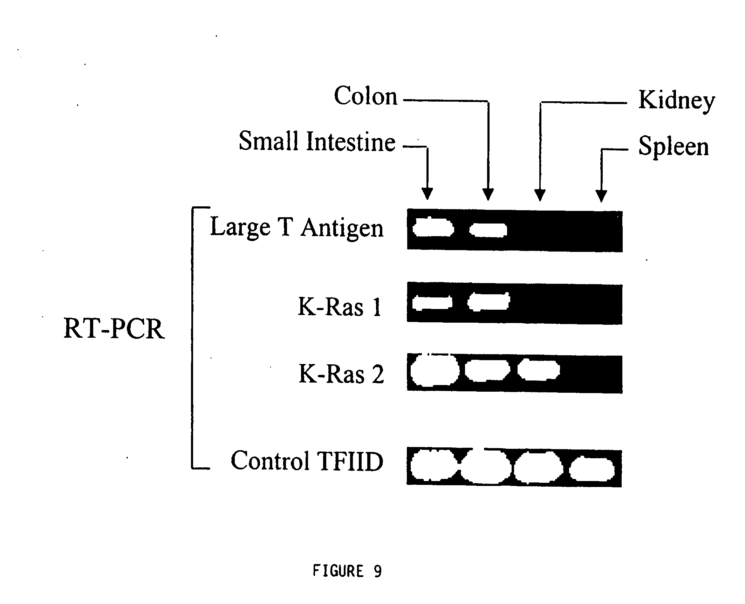

Several of these applications are illustrated in the proposed protocols disclosed on Figure 8.

### II Obtention of the 9 kb regulatory sequences from the murine villin gene associated with mutated K-ras (val 12)

### II-1 Obtention of the vector p(murine villin 9kb) AatII

The vector p2kb/lacZ was constructed like the plasmids illustrated on figure 3 except that a 2kb fragment containing the 3' portion of the 9kb villin gene beginning at the BamH1 site (located at nucleotide 7076) and ending at the ATG corresponding to the Ncol site, was used for the association to LacZ.

### A NotI site was introduced into the vector p2kb/lacZ using a linker SpeI→NotI:

CTAGGCGGCCGC (→p2kb/LacZ ^{Spe/Not}). Digestion by NcoI, NotI eliminated the LacZ gene. The NcoI site at the ATG from the villin gene was converted to AatII in the vector containing 2kb 3' of the regulatory sequences of the villin gene by using hybridized oligonucleotides (5' CATGACGTCGGACTTGC and 3' GGCCGCAAGTCCGACGT). A fragment AfIII-NotI comprising the 3' end of the regulatory sequence of the villin gene and the AatII site,was then introduced in the vector p9kb/LacZ digested by AfIII and Not I, thereby replacing the LacZ portion. The plasmid p(murine villin 9kb) AatII is thus obtained.

### II-2 Preparation of the p(murine villin 9kb) AatII for ligation with the Kras fragment

The p(murine villin 9kb) AatII is digested by AatII and NotI.

### II-3 The K-ras gene

The K-ras mutated in Val 12 (Mc Coy M et al (1984) Human colon carcinoma Ki-ras2 oncogene and its corresponding proto-oncogene, Mol. Cell. Biol. 4, 1577-1582) was amplified by PCR using an sens oligonucleotide K-ras containing a ScaI site (TGCAAAAGTACTGAATATAAACTTGTG) and an antisens K-ras containing a NotI site (ATTTGCGGCCGCTTTACATAATTACACACT).

### II-4 The resulting fragment (500bp is inserted in the digested p(murine villin 9kb)

AatII to prepare the p(murine villin 9kb AatII/K-ras (val12).

For transgenesis, the transgene was exised by KpnI and NotI.

### III Obtention of the 9 kb regulatory sequences from the murine villin gene associated to T/t tsA58

### 111-1 Subcloning T/t tsA58 into ks

The 5' region of the T/t tsA58 gene (Jat, P.S. and P.A. Sharp. 1989. Cell lines established by a temperature-sensitive simian virus 40 large T-antigen are growth restricted at the non permissive temperature. Mol Cell. Biol. 9. 1672-1681) was amplified by PCR using Oligonucleotides (5' GGGTACCATGGATAAAGTTTTAAACAGAGAG and 3' GGAATTCGGCGCCGCAGTAGCAATCAACCC) creating a KpnI-NcoI site at the 5' end and a EcoRI-NarI site at the 3'end. The EcoRI/KpnI fragment was introduced into Bluescript ks (→pks T/t 5'). This plasmid was then digested by NarI/Smal, insert the 3' fragment of T/t tsA58, also used as template for PCR digested ClaI/Klenow/TaqI (→ pKS T/t tsA58).

### III-2 Cloning T/t behind 2kb-fragment of the villin promoter

A NotI site was introduced into the vector p2kb/lacZ described above using a linker

SpeI→NotI: CTAGGCGGCCGC (→p2kb/LacZ ^{Spe/Not}). The T/t tsA58 digested by NcoI/NotI was introduced in the p2kb/ LacZ ^{Spe/Not} replacing the LacZ portion.

### III-3 Cloning T/t tsA58 + 293bp of villin promoter (→AfIII site) into p9kb/lacZ

Cloning 2kb/T/t tsA58 AfIII/NotI into p9kb/LacZ
(→p(murine villin 9kb) T/t tsA58)

For transgenesis, the transgene was exised by KpnI and NotI.

### REFERENCES

1. Sweetser, D. A., Hauft, S. M., Hoppe, P. C., Birkenmeier, E. H. and Gordon, J. I. (1988) Proc. Natl. Acad Sci. USA 85, 9611-9615
2. Cohn, S. M., Simon, T. C., Roth, K. A., Birkenmeier, E. H. and Gordon, J. I. (1992) J. Cell Biol. 119,27-44
3. Hermiston, M. L., Green, R. P. and Gordon, J. 1. (1993) Proc. Natl. Acad Sci. USA 90, 8866-8870
4. Markowitz, A. J., Wu, G. D., Birkenmeier, E. H. and Traber, P. G. (1993) Am. J Physiol. 265, G526-G539
5. Crossman, M. W., Hauft, S. M. and Gordon, J. I. (1994) J. Cell Biol. 126, 1547-1564
6. Cheng, H. and Leblond, C. P. (1974) Am. J. Anat. 141, 461-479
7. Wright, N. A. and Irwin, M. (1982) Cell Tiss. Kinet. 15, 595-609
8. Gordon, J. I. and Hermiston, M. L. (1994) Curr. Opin. Cell Biol. 6, 795-803
9. Ponder, B. A., Schmidt, G. H., Wilkinson, M. M., Wood, M. J., Monk, M. and Reid, A. (1985) Nature (London) 313, 689-691
10. Potten, C. S. and Loeffler, M. (1990) Development (Cambridge, U.K.) 110, 1001- 1020
11. Schmidt, G. H., Wilkinson, M. M. and Ponder, B. A. (1985) Cell 40, 425-429.
12. Hall, P. A., Coates, P. J., Ansari, B. and Hopwood, D. (1994) J Cell Sci. 107, 3569-3577
13. Bry, L., Falk, P., Huttner, K., Ouellette, A., Midtvedt, T. and Gordon, J. I. (1994) Proc. Natl. Acad. Sci. USA 91, 10335-10339
14. Hauft, S. M., Kim, S. H., Schmidt, G. H., Pease, S., Rees, S., Harris, S., Roth, K. A., Randall Hansbrough, J., Cohn, S. M., Ahnen, D. J., Wright, N. A., Goodlad, R. A. and Gordon, J. I. (1992) J Cell Biol. 117, 825-839
15. Kim, S. H., Roth, K. A., Moser, A. R. and Gordon, J. I. (1993) J Cell Biol. 123, 877-893
16. Robine, S., Huet, C., Moll, R., Sahuquillo-Merino, C., Coudrier, E., Zweibaum, A. and Louvard, D. (1985) Proc. Acad Natl. Sci. USA 82, 8488-8492
17. Boller, K., Arpin, M., Pringault, E., Mangeat, P. and Reggio, H. (1988) Differentiation 39, 51-57
18. Maunoury, R., Robine, S., Pringault, E., Huet, C., Guenet, J. L., Gaillard, J. A. and Louvard, D. (1988) EMBOJ. 7, 3321-3329
19. Maunoury, R., Robine, S., Pringault, E., Leonard, N., Gaillard, J. A. and Louvard, D. (1992) Development (Cambridge, U.K) 115, 717-728
20. Ezzell, R. M., Chafel, M. M. and Matsudaira, P. T. (1989) Development (Cambridge, U.K.) 106, 407-419
21. Carboni, J. M., Howe, C. L., West, A. B., Barwick, K. W., Mooseker, M. S. and Morrow, J. S. (1987) Am. J. Pathol. 129, 589-600
22. Moll, R., Robine, S., Dudouet, B. and Louvard, D. (1987) Virchows Arch. 54, 155- 169
23. West, A. B., Isaac, C. A., Carboni, J. M., Morrow, J. S., Mooseker, M. S. and Barwick, K. W. (1988) Gastroenterology 94, 343-352
24. Bacchi, C. E. and Gown, A. M. (1991) Lab. Invest. 64, 418-424
25. Pringault, E., Robine, S. and Louvard, D. (1991) Proc. Natl. Acad. Sci. USA 88, 10811-10815
26. Robine, S., Sahuquillo-Merino, C., Louvard, D. and Pringault, E. (1993) J. Biol. Chem. 268, 11426-11434
27. Green, R. P., Cohn, S. M., Sacchettini, J. C., Jackson, K. E. and Gordon, J. I. (1992) DNA Cell Biol. 11, 31-41
28. Perret, C., L'Horset, F. and Thomasset, M. (1991) Gene 108, 227-235
29. Cohen-Tannoudji, M., Robine, S., Choulika, A., Pinto, D., El Marjou, F., Babinet, C., Louvard, D. and Jaisser, F. (1998) Mol. Cell. Biol. 18, 1444-1448
30. Breathnach, R. and Chambon, P. (1981) Annu. Rev. Biochem. 50, 349-83.
31. Becker, P. B. (1994) BioEssays 16, 541-547
32. Robine, S., Jaisser, F. and Louvard, D. (1997) Am. J Physiol. 273, G759-G762
33. Cui, C., Wani, M. A., Wight, D., Kopchick, J. and Stambrook, P. J. (1994) Transgenic Res. 3, 182-194
34. Aronow, B. J., Silbiger, R. N., Dusing, M. R., Stock, J. L., Yager, K. L., Potter, S. S., Hutton, J. J. and Wiginton, D. A. (1992) Mol. Cell. Biol. 12,4170-4185
35. Sabourin, J. C., Kern, A. S., Gregori, C., Porteu, A., Cywiner, C., Chatelet, F. P., Kahn, A. and Pichard, A. L. (1996) J. Biol. Chem. 271, 3469-3473
36. Hermiston, M. L. and Gordon, J. I. (1995) J. Cell Biol. 129, 489-506
37. Rottman, J. N. and Gordon, J. 1. (1993) J. Biol. Chem. 268, 11994-12002
38. Bisaha, J. G., Simon, T. C., Gordon, J. I. and Breslow, J. L. (1995) J. Biol. Chem. 270, 19979-19988
39. Simon, T. C., Roberts, L. J. and Gordon, J. I. (1995) Proc. Natl. Acad Sci. USA 92, 8685-8689
40. Simon, T. C., Cho, A., Tso, P. and Gordon, J. I. (1997) J. Biol. Chem. 272, 10652-10663
41. Fearon, E. R. and Vogelstein, B. (1990) Cell 61, 759-767
42. Efrat, S., Linde, S., Kofod, H., Spector, D., Delannoy, M., Grant, S., Hanahan, D. and Baekkeskov, S. (1988) Proc. Natl. Acad. Sci. USA 85, 9037-9041
43. Hanahan, D. (1988) Ann. Rev. Genet. 22, 479-519
44. Cartier, N., Lacave, R., Vallet, V., Hagege, J., Hellio, R., Robine, S., Pringault, E., Cluzeaud, F., Briand, P. and Kahn, A. (1993)J. Cell Sci. 104, 695-704.

## Claims

1. Nucleotide sequence derived from the 5' sequence of the murine villin gene, having a size of 9 kb on an agarose gel, and comprising SEQ ID NO: 1, or a fragment thereof, comprising the nucleotides elements having a cis-regulatory activity that promotes the transcription of the murine villin gene, and having retained the transcription initiation site of the murine villin gene.

2. Nucleotide sequence according to Claim 1, which is the sequence extending 3.5 kb upstream and 5.5 kb downstream from the transcription initiation site of the murine villin gene.

3. Nucleotide sequence according to claim 1 which is the sequence identified as Seq ID NO: 1.

4. Nucleotide sequence according to anyone of claims 1 to 3, which comprises the nucleotide fragment extending from the HS I to the HS IV Dnase I-hypersensitive sites.

5. Nucleotide sequence according to anyone of claims 1 to 4, comprising the nucleotide fragment extending from the HS IV Dnase-hypersensitive site to the translation initiation site of the murine villin gene.

6. Nucleotide sequence according to claim 1, which comprises the nucleotide fragment extending from the nucleotide at around position -100 upstream from the transcription initiation site, to the translation initiation site.

7. Nucleotide sequence according to claim 1, which comprises the nucleotide fragment extending from around the nucleotide at position -480 from the transcription initiation site, to the translation initiation site.

8. Nucleotide sequence according to claim 1, which is the sequence extending 3.5 kb upstream from the transcription initiation site to the translation initiation site, provided the region corresponding to intron 1, located between said sites is deleted, or deleted in part.

9. Nucleotide sequence according to anyone of claims 1 to 7, which comprises nucleotide regions having a regulatory activity affecting the level of expression of the murine villin gene.

10. Nucleotide sequence according to claim 1, which has a regulatory activity on the level of expression of the murine villin gene in intestine cells and/or in transgenic mice.

11. Recombinant nucleotide sequence comprising a first nucleotide sequence according to anyone of claims 1 to 10 and a second nucleotide sequence for which a tissue specific targeted expression in epithelial intestine cells is sought.

12. Recombinant nucleotide sequence according to claim 11, wherein the second nucleotide sequence is a sequence encoding a determined polypeptide.

13. Recombinant nucleotide sequence according to anyone of claims 11 to 12, wherein the second nucleotide sequence is a sequence of therapeutic interest.

14. Recombinant nucleotide sequence according to anyone of claims 11 to 13, wherein the second nucleotide sequence is an oncogene.

15. Recombinant nucleotide sequence according to claim 11, wherein the second nucleotide sequence is a tumor suppressor gene.

16. Recombinant nucleotide sequence according to claim 11, wherein the second nucleotide sequence encodes an immunoglobulin or a fragment thereof, especially a variable fragment thereof

17. Recombinant nucleotide sequence according to anyone of claims 11 to 16 which further comprises a third nucleotide sequence consisting of a reporter sequence under the control of said first nucleotide sequence.

18. Recombinant nucleotide sequence according to anyone of claims 11 to 17 wherein the second nucleotide sequence is placed under the control of an inducible system.

19. Recombinant cell comprising a recombinant sequence according to anyone of claims 11 to 18.

20. Recombinant cell according to claim 19, which is an epithelial cell originating from the intestinal tract.

21. Recombinant cell according to claim 20, which is a epithelial stem cell.

22. Recombinant cell according to claim 20, which is a differentiated cell.

23. Recombinant cell according to claim 19, which is an epithelial cell originating from the kidney proximal tubules.

24. Recombinant epithelial cell according to anyone of claims 20 to 23 which is immortalized.

25. Trangenic non-human animal expressing a recombinant nucleotide sequence according to anyone of claims 11 to 18.

26. Transgenic animal according to claim 25 which is a Vertebrate especially a non human mammal, a bird or a fish or which is an Invertebrate especially Drosophila or a Nematode like *C. elegans.*

27. Transgenic animal according to claim 26 which is a mouse.

28. Process for the preparation of transgenic mice comprising the steps of:
- administration of a recombinant nucleotide sequence according to any one of claims 11-18 into the pronuclei of fertilized eggs of mice,
- enabling the development of the recombined eggs to recover transgenic mice (founders) and verifying the presence of the transgene,
- if appropriate crossing the founders with non transgenic mice.

## Patentansprüche

1. Nucleotidsequenz abgeleitet aus der 5'-Sequenz des murinen Villin-Gens, die auf einem Agarosegel eine Größe von 9 kb aufweist und SEQ ID NO: 1 oder ein Fragment davon umfasst, die die Nucleotidelemente mit cis-regulatorischer Aktivität umfasst, die die Transkription des murinen Villin-Gens fördert, und in der die Transkriptionsinitiationsstelle des murinen Villin-Gens erhalten ist.

2. Nucelotidsequenz nach Anspruch 1, die die Sequenz ist, die sich 3,5 kb stromaufwärts und 5,5 kb stromabwärts der Transkriptionsinitiationsstelle des murinen Villin-Gens erstreckt.

3. Nucleotidsequenz nach Anspruch 1, die die als SEQ ID NO: 1 identifizierte Sequenz ist.

4. Nucleotidsequenz nach einem der Ansprüche 1 bis 3, die das Nucleotidfragment umfasst, das sich von den HS-I- bis zu den HS-IV-DNase-hypersensitiven Stellen erstreckt.

5. Nucleotidsequenz nach einem der Ansprüche 1 bis 4, umfassend das Nucleotidfragment, das sich von der HS-IV-DNase-hypersensitiven Stelle bis zur Translationsinitiationsstelle des murinen Villin-Gens erstreckt.

6. Nucleotidsequenz nach Anspruch 1, die das Nucelotidfragment umfasst, das sich vom Nucleotid an etwa Position -100 stromaufwärts der Transkriptionsinitiationsstelle bis zur Translationsinitiationsstelle erstreckt.

7. Nucleotidsequenz nach Anspruch 1, die das Nucleotidfragment umfasst, das sich von etwa dem Nucleotid an Position -480 von der Transkriptionsinitiationssequenz bis zur Translationsinitiationsstelle erstreckt.

8. Nucleotidsequenz nach Anspruch 1, die die Sequenz ist, die sich 3.5 kb stromaufwärts von der Transkriptionsinitiationsstelle bis zur Translationsinitiationsstelle erstreckt, mit der Maßgabe, dass die dem Intron 1 entsprechende Region, die zwischen diesen Stellen liegt, deletiert oder teilweise deletiert ist.

9. Nucleotidsequenz nach einem der Ansprüche 1 bis 7, die Nucleotidregionen umfasst, die eine den Expressionsgrad des murinen Villin-Gens beeinflussende regulatorische Aktivität aufweisen.

10. Nucleotidsequenz nach Anspruch 1, die eine regulatorische Aktivität hinsichtlich des Expressionsgrades des murinen Villin-Gens in Darmzellen und/oder in transgenen Mäusen aufweist.

11. Rekombinante Nucleotidsequenz, umfassend eine erste Nucleotidsequenz nach einem der Ansprüche 1 bis 10 und eine zweite Nucleotidsequenz, für die eine gewebespezfische zielgerichtete Expression in epithelialen Darmzellen angestrebt wird.

12. Rekombinante Nucleotidsequenz nach Anspruch 11, wobei die zweite Nucleotidsequenz eine ein bestimmtes Polypeptid codierende Sequenz ist.

13. Rekombinante Nucleotidsequenz nach einem der Ansprüche 11 bis 12, wobei die zweite Nucleotidsequenz eine Sequenz von therapeutischem Interesse ist.

14. Rekombinante Nucleotidsequenz nach einem der Ansprüche 11 bis 13, wobei die zweite Nucleotidsequenz ein Oncogen ist.

15. Rekombinante Nucleotidsequenz nach Anspruch 11, wobei die zweite Nucleotidsequenz ein Tumorsuppressorgen ist.

16. Rekombinante Nucelotidsequenz nach Anspruch 11, wobei die zweite Nucleotidsequenz ein Immunoglobulin oder ein Fragment davon, insbesondere ein variables Fragment davon codiert.

17. Rekombinante Nucleotidsequenz nach einem der Ansprüche 11 bis 16, die ferner eine dritte Nucleotidsequenz umfasst, die aus einer Reportersequenz unter der Kontrolle der ersten Nucleotidsequenz besteht.

18. Rekombinante Nucleotidsequenz nach einem der Ansprüch 11 bis 17, wobei die zweite Nucleotidsequenz unter die Kontrolle eines induzierbaren Systems gestellt ist.

19. Rekombinante Zelle, umfassend eine rekombinante Sequenz nach einem der Ansprüche 11 bis 18.

20. Rekombinante Zelle nach Anspruch 19, die eine aus dem Instestinaltrakt stammende Epithelzelle ist.

21. Rekombinante Zelle nach Anspruch 20, die eine epitheliale Stammzelle ist.

22. Rekombinante Zelle nach Anspruch 20, die eine differenzierte Zelle ist.

23. Rekombinante Zelle nach Anspruch 19, die eine aus den proximalen Nierentubuli stammende Epithelzelle ist.

24. Rekombinante Epithelzelle nach einem der Ansprüche 20 bis 23, die immortalisiert ist.

25. Transgenes nicht-menschliches Tier, das eine rekombinante Nucleotidsequenz nach einem der Ansprüche 11 bis 18 exprimiert.

26. Transgenes Tier nach Anspruch 25, das ein Wirbeltier, insbesondere ein nicht-menschliches Säugetier, ein Vogel oder ein Fisch ist, oder ein Invertebrat, insbesondere Drosophila oder ein Nematode wie C. *elegans* ist.

27. Transgenes Tier nach Anspruch 26, das eine Maus ist.

28. Verfahren zur Herstellung transgener Mäuse, umfassend die Schritte :
- Einbringen einer rekombinanten Nucleotidsequenz nach einem der Ansprüche 11 bis 18 in die Pronuclei befruchteter Eizellen von Mäusen,
- Aktivieren der Entwicklung der rekombinierten Eizellen, um transgene Mäuse (Founder) zu erhalten, und Überprüfen der Anwesenheit des Transgens,
- gegebenenfalls Kreuzen der Founder mit nicht-transgenen Mäusen.

## Revendications

1. Séquence nucléotidique dérivée de la séquence en 5' du gène murin de la villine, ayant une taille de 9 kb sur un gel d'agarose, et comprenant SEQ ID NO : 1, ou un fragment de celle-ci, comprenant les éléments nucléotidiques possédant une activité cis-régulatrice qui promeut la transcription du gène murin de la villine, et ayant conservé le site d'initiation de la transcription du gène murin de la villine.

2. Séquence nucléotidique selon la revendication 1, qui est la séquence s'étendant sur 3,5 kb en amont et 5,5 kb en aval du site d'initiation de la transcription du gène murin de la villine.

3. Séquence nucléotidique selon la revendication 1, qui est la séquence identifiée comme SEQ ID NO : 1.

4. Séquence nucléotidique selon l'une quelconque des revendications 1 à 3, qui comprend le fragment nucléotidique s'étendant du site hypersensible à la DNase I HS I au site hypersensible à la DNase I HS IV.

5. Séquence nucléotidique selon l'une quelconque des revendications 1 à 4, comprenant le fragment nucléotidique s'étendant du site hypersensible à la DNase I HS IV au site d'initiation de la traduction du gène murin de la villine.

6. Séquence nucléotidique selon la revendication 1, qui comprend le fragment nucléotidique s'étendant du nucléotide autour de la position -100 en amont du site d'initiation de la transcription au site d'initiation de la traduction.

7. Séquence nucléotidique selon la revendication 1, qui comprend le fragment nucléotidique s'étendant à peu près du nucléotide à la position -480 par rapport au site d'initiation de la transcription au site d'initiation de la traduction.

8. Séquence nucléotidique selon la revendication 1, qui est la séquence s'étendant de 3,5 kb en amont du site d'initiation de la transcription au site d'initiation de la traduction, à condition que la région correspondant à l'intron 1 située entre lesdits sites soit délétée, ou partiellement délétée.

9. Séquence nucléotidique selon l'une quelconque des revendications 1 à 7, qui comprend des régions nucléotidiques possédant une activité régulatrice affectant le niveau d'expression du gène murin de la villine.

10. Séquence nucléotidique selon la revendication 1, qui possède une activité régulatrice sur le niveau d'expression du gène murin de la villine dans des cellules intestinales et/ou chez des souris transgéniques.

11. Séquence nucléotidique recombinante comprenant une première séquence nucléotidique selon l'une quelconque des revendications 1 à 10 et une deuxième séquence nucléotidique pour laquelle une expression ciblée spécifique d'un tissu dans des cellules épithéliales intestinales est recherchée.

12. Séquence nucléotidique recombinante selon la revendication 11, dans laquelle la deuxième séquence nucléotidique est une séquence codant un polypeptide déterminé.

13. Séquence nucléotidique recombinante selon l'une quelconque des revendications 11 à 12, dans laquelle la deuxième séquence nucléotidique est une séquence d'intérêt thérapeutique.

14. Séquence nucléotidique recombinante selon l'une quelconque des revendications 11 à 13, dans laquelle la deuxième séquence nucléotidique est un oncogène.

15. Séquence nucléotidique recombinante selon la revendication 11, dans laquelle la deuxième séquence nucléotidique est un gène suppresseur de tumeur.

16. Séquence nucléotidique recombinante selon la revendication 11, dans laquelle la deuxième séquence nucléotidique code une immunoglobuline ou un fragment de celle-ci, en particulier un fragment variable de celle-ci.

17. Séquence nucléotidique recombinante selon l'une quelconque des revendications 11 à 16, qui comprend en outre une troisième séquence nucléotidique consistant en une séquence rapporteur sous le contrôle de ladite première séquence nucléotidique.

18. Séquence nucléotidique recombinante selon l'une quelconque des revendications 11 à 17, dans laquelle la deuxième séquence nucléotidique est placée sous le contrôle d'un système inductible.

19. Cellule recombinante comprenant une séquence recombinante selon l'une quelconque des revendications 11 à 18.

20. Cellule recombinante selon la revendication 19, qui est une cellule épithéliale issue du tractus intestinal.

21. Cellule recombinante selon la revendication 20, qui est une cellule souche épithéliale.

22. Cellule recombinante selon la revendication 20, qui est une cellule différenciée.

23. Cellule recombinante selon la revendication 19, qui est une cellule épithéliale issue des tubules proximaux du rein.

24. Cellule épithéliale recombinante selon l'une quelconque des revendications 20 à 23, qui est immortalisée.

25. Animal transgénique non humain exprimant une séquence nucléotidique recombinante selon l'une quelconque des revendications 11 à 18.

26. Animal transgénique selon la revendication 25, qui est un vertébré, en particulier un mammifère non humain, un oiseau ou un poisson ou qui est un invertébré, en particulier *Drosophila* ou un nématode comme *C*. *elegans.*

27. Animal transgénique selon la revendication 26, qui est une souris.

28. Procédé de préparation de souris transgéniques, comprenant les étapes consistant à :
- administrer une séquence nucléotidique recombinante selon l'une quelconque des revendications 11 à 18 dans les pronoyaux d'oeufs fertilisés de souris,
- permettre le développement des oeufs recombinés pour recueillir des souris transgéniques (fondatrices) et vérifier la présence du transgène,
- si approprié, croiser les fondatrices avec des souris non transgéniques.
